# EUROPEAN PATENT APPLICATION

(11) **EP 2 409 669 A1**
(43) Date of publication of application: **25.01.2012**
(21) Application number: 11185474.1
(22) Date of filing: 31.07.2007
(51) Int. Cl.: A61F 2/12, A61K 35/38, A61F 2/02

(54) **Mastopexy and breast reconstruction prostheses**

(30) Priority: 31.07.2006 US 820905 P
(62) Divisional of application: 07813589.4
(71) Applicant: Organogenesis, Inc., Canton, MA 02021 (US)
(72) Inventor: The designation of the inventor has not yet been filed
(74) Representative: Leonard, Thomas Charles

(57) **Abstract**

Mastopexy and breast reconstruction prostheses and implantation method that allow for radiographic imaging of the breast tissue. The prostheses are arcuate and elongate optionally meshed to conform with breast tissue when implanted. Prostheses are made from naturally occurring extracellular matrix, primarily collagen, that, allows for mammographic imaging without interference as is expected from synthetic materials.

## Description

### Cross-Reference to Related Application

This application claims the benefit of priority to U.S. Provisional Application 60/820,905, filed July 31, 2006, which application is hereby incorporated herein by reference in its entirety.

### Field of the Invention

This invention is in the field of tissue engineering. The invention is directed to bioengineered graft prostheses prepared from cleaned tissue material derived from animal sources. The bioengineered graft prostheses of the invention are prepared using methods that preserve biocompatibility, cell compatibility, strength, and bioremodelability of the processed tissue matrix. The bioengineered graft prostheses are used for implantation, repair, or for use in a mammalian host.

### Brief Description of the Background of the Invention

The field of tissue engineering combines the methods of engineering with the principles of life science to understand the structural and functional relationships in normal and pathological mammalian tissues. The goal of tissue engineering is the development and ultimate application of biological substitutes to restore, maintain, and improve tissue functions.

Collagen is the principal structural protein in the body and constitutes approximately one-third of the total body protein. It comprises most of the organic matter of the skin, tendons, bones, and teeth and occurs as fibrous inclusions in most other body structures. Some of the properties of collagen are its high tensile strength; its low antigenicity, due in part to masking of potential antigenic determinants by the helical structure; and its low extensibility, semipermeability, and solubility. Furthermore, collagen is a natural substance for cell adhesion. Collagen-based materials are bioremodelable provided that they are mechanically and chemically processed in a way that preserves bioremodelability in contrast to synthetic materials where a lack of bioremodelability is a drawback. These properties and others make collagen a suitable material for tissue engineering and manufacture of implantable biocompatible substitutes and bioremodelable prostheses.

Methods for obtaining collagenous tissue and tissue structures from explanted mammalian tissues and processes for constructing prosthesis from the tissue, have been widely investigated for surgical repair or for tissue or organ replacement. It is a continuing goal of researchers to develop prostheses that can successfully be used to replace or repair mammalian tissue.

There is a need for collagen-based materials and prostheses for use in procedures impacting human breast tissue. In recent years, the rate of plastic surgery procedures has increased and many women elect to have surgery to change the size, shape, position of their breasts. As a separate but related matter, the rate of breast reconstruction surgeries that follow mastectomy procedures have increased as cancer detection methods have improved and as many women monitor breast health more closely.

Mastopexy, or breast lift, is a procedure designed to improve the appearance of sagging or ptotic breasts. The goal of surgery is to improve the shape and position (i.e. lift) of the breast while minimizing visible scars. To achieve this end result, multiple procedures and countless modifications of the mastopexy have been suggested.

While descriptions of reduction mammoplasties can be seen as early as Paulus of Aegina (625-690 AD), not until the late 19th century was emphasis placed on correcting ptosis of the breast. Much of the history of mastopexy parallels that of breast reduction, since both attempt to alter the shape of the breast and the skin envelope. Most of these procedures involved elevation of the breast mound using suspension techniques.

Techniques that transposed the nipple-areola complex (NAC) as a vascular pedicle were described by Morestin (1907) and used by Lexer (1912). Thorek (1921) was credited with the first report of a free nipple graft. Hollander (1924) first reported the lateral oblique resection resulting in an L-shaped scar. Schwarzmann (1937) described the use of periareolar de-epithelialization to preserve the neurovascular supply of the NAC. By the 1930s, most of the essential technical elements of the mastopexy had been developed.

Further evolution in the mastopexy resulted in refinement of technique and analysis. Aufricht (1949) advocated preoperative planning using a geometric system and stressed the concept of the skin envelope defining the final breast shape. Wise (1956) defined the preoperative geometric marking system most commonly used today. Gonzalez-Ulloa (1960) first advocated mastopexy with augmentation for ptosis with hypoplasia or atrophy. Goulian (1971) described the use of the dermal mastopexy, and Regnault (1976) presented a classification system for breast ptosis and a description of the B mammoplasty.

Johnson (1981), among others, has used Marlex mesh to lift the breast parenchyma. Benelli (1990) reported the use of the periareolar round block or purse string mammoplasty. Procedures to recreate breast fullness using autologous tissue either primarily or after breast prosthesis explantation have been described by Weiss and Ship (1995) and Flowers (1998). Hall-Findley (1999) used a medial-based pedicle modification of the vertical scar approach first described by Lassus (1970) as superior pedicle and popularized by Lej our (1994) with the use of breast liposuction.

Mastopexy presents one of the greatest challenges to the breast surgeon but previous techniques have drawbacks. Numerous techniques provide improvement in the shape of the breast. The aesthetic goals of these techniques are to obtain a more youthful appearance, improved projection, and reduced ptosis but aesthetic improvement comes at the cost of scars. In addition, although breast implants can provide the upper pole projection patients often desire, they present specific risks and complications.

While the incidence of breast ptosis is difficult to estimate, the frequency of mastopexy clearly is increasing. The American Society of Plastic Surgeons reported a 509% increase in procedures from 1997 to 2005.

Etiology is varied and can be due to several components but gravity seems to be a common factor. Younger patients are more prone to ptosis because of excessive breast size or thin skin, thus the intertwining of breast reduction and mastopexy procedures. Ptosis in middle-aged patients usually is due to postpartum changes; the breast skin is stretched during lactation or engorgement, and afterward the breast gland atrophies, leaving loosened skin. Finally, in postmenopausal patients, further atrophy, gravity, loss of skin elasticity due to age, and weight gain are factors in creating breast ptosis.

With time, relaxation of Cooper ligaments and dermal laxity cause descent of the breast tissue and NAC. Postpartum involutional changes exacerbate the laxity of the suspensory ligaments and skin envelope. To properly correct these changes, elevating the breast parenchyma is necessary. In addition, the redundant skin envelope must be removed and the NAC must be transposed.

In most instances, breast mastopexy has no true medical indications and is performed primarily for aesthetic reasons. The main exception to this is in postmastectomy reconstruction, when performing a mastopexy often is essential to achieving symmetry. Another indication is following implant removal, which can result in breast ptosis and lax skin. However, one must be careful in assessing the amount of ptosis in patients with breast implants that are contracted and high riding.

Four main types of breast lifts exist, and the common names of them are based on the shape of the incision and resulting scar. The more sagging a patient has, the more likely that she will need more extensive and longer incisions to achieve a desirable result. With any of these techniques, the nipple and areola complex can be shifted to either side as well as up, if necessary, for the most aesthetic appearance. A breast lift does not involve removal and replacement of the nipple. The nipple and areola stay attached to the breast, and only surrounding skin is removed. A summary of common techniques follows:

Crescent mastopexy - For patients with mild sagging, excess breast skin in the upper half of the breast, and a normal amount of skin in the lower half, a semi-circular incision is made on the upper portion of the areola. A crescent shaped piece of skin is removed, and when the skin edges are sewn back together, the nipple and areola are raised slightly (1 to 2 inches). A crescent mastopexy is best for women with only mild breast ptosis (sagging).

Donut mastopexy - Also called a Benelli mastopexy or circumareolar mastopexy since the incision is around the areola, a donut mastopexy removes a ring of skin from outside the areola. Sutures are then placed around the areola and the skin is tightened like a purse string to lift the breast. Puckering of the skin may occur, and usually resolves on its own within a few months. The donut mastopexy is also useful for women with a projecting nipple/areola complex (sometimes called torpedo or missile shaped breasts), and can also be used to reduce the size of the areola at the same time.

Lollipop or vertical mastopexy - As the name implies, the incision for a lollipop mastopexy is made around the areola and then down the center of the breast to the inframammary fold. This technique is used for mild to moderate breast ptosis. As with the circumareolar or donut lift, the size of the areola may be reduced at the same time.

Anchor mastopexy - Also referred to as a Wise pattern (or sometimes Weiss pattern) mastopexy, full breast lift, or inverted-T incision, the anchor mastopexy is considered the traditional technique for breast lifting. The incisions are made around the areola, down the center of the lower portion of the breast and then across the breast in the inframammary fold. Like the donut and lollipop incisions, the areola can be made smaller at the same time. The resulting scar is in the shape of an anchor. Although the Wise pattern or anchor mastopexy used to be the standard, it is now usually reserved only for those with moderate to severe breast sagging.

Breast reconstruction is the re-creation of a breast following mastectomy. Mastectomy is the most common treatment of localized breast cancer but may negatively impact the patient emotionally, leaving her feeling deformed and mutilated, leading to anger, depression, and anxiety. While breast reconstruction can be performed at the time of mastectomy, the better candidates are those who have confirmed elimination of the cancer as sometimes implant materials and reconstruction will interfere with detection of recurrence. Reconstruction usually involves a two part process, where in the first series of surgeries, a tissue expander is inserted beneath the skin and the pectoralis muscle. The expander is an air or saline-filled balloon that is periodically injected over a number of months with additional saline in order to gradually stretch the skin and muscle. When the skin and muscle are sufficiently lengthened, an implant (saline or silicone) is inserted to recapitulate the native breast structure. However, in order to retain the implant properly, an additional section of a patient's tissue, an autograft, must be used along the lateral side of the breast, usually the latissimus dorsi or abdominus recti. Autograft tissue bears a risk of tissue morbidity and total coverage and support of the implant or the expander with the muscle tissue in the mastectomy pocket is a challenge. Without appropriate coverage, the implant can become exposed and reduce cosmetic outcome. For these patients, a ready-to-use, off-the-shelf prosthesis made from a material compatible with cell and tissue is needed to both cover and support the implant or the expander at the lower breast pole.

Heretofore, mastopexy and breast reconstruction materials and prostheses fabricated from biosynthetic materials and methods for their implantation have drawbacks in that they interfere with mammographical imaging that is necessary for detecting breast tissue abnormalities, including cancerous tumors. The reason for their interference is in that these prostheses are fabricated from materials that are not found in human or animal tissues and so they are evidenced in mammography and obscure imaging of the breast tissue. Because these mastopexy devices cup a significant portion of the round of the breast, previous implant methods excessively disrupt the breast tissue by separating the tissue layers and cause a slow healing response and create a potential risk for tissue morbidity. Materials derived from human cadaver tissue, usually from skin, also offer drawbacks in that their supply is limited and reports have demonstrated that their sourcing has been met with ethical challenges and safety concerns.

There is a need for collagen-based materials and prostheses that are thin but strong so as to perform a tissue support function over time but are thin so as not to interfere with other tissue structures or with the cosmetic outcome when implanted in soft-tissue. There is also a need for a bioremodelable collagen-based material which, when implanted into the body, will undergo controlled biodegradation occurring concomitantly with remodeling and replacement by a patient's own cells and tissue matrix, while providing the requisite support and shape of the tissue that it is replacing or repairing. There is a further need for collagen-based materials that are procured from a traceable source and are purified so as to mitigate ethical and safety concerns that are present in materials derived from cadavers. There is a still further need for materials and prostheses in mastoxpexy procedures and breast reconstruction that do not interfere with mammography and monitoring of breast health.

The present invention addresses these drawbacks and unmet needs to advance the treatment standards for mastopexy treatments and breast reconstruction with a novel mastopexy prosthesis and implantation method and a breast reconstruction prosthesis.

### SUMMARY OF THE INVENTION

Biologically-derived collagenous materials such as the intestinal submucosa have been proposed by many investigators for use in tissue repair or replacement. Methods for mechanical and chemical processing of the proximal porcine jejunum to generate a single, acellular layer of intestinal collagen (ICL) that can be used to form laminates for bioprosthetic applications are disclosed. The processing removes cells and cellular debris while maintaining the native collagen structure. The resulting sheet of processed tissue matrix is used to manufacture multi-layered laminated constructs with desired specifications. We have investigated the efficacy of laminated patches for soft tissue repair as well as the use of entubated ICL as component of a vascular graft. This material provides the necessary physical support, while generating minimal adhesions and is able to integrate into the surrounding native tissue and become infiltrated with host cells. *In vivo* remodeling does not compromise mechanical integrity. Intrinsic and functional properties of the implants made from ICL, such as the modulus of elasticity, suture retention and ultimate tensile strength are important parameters which can be manipulated for specific requirements by varying the number of ICL layers and the crosslinking conditions.

It is an object of the invention to provide a prosthesis for mastopexy or breast reconstruction procedures comprising processed tissue material which, when implanted into a mammalian patient, undergoes controlled biodegradation occurring with adequate living cell replacement such that the original implanted prosthesis is remodeled by the patient's living cells; wherein the processed tissue material is derived from intestine or dermis; wherein the prosthesis has an arcuate or crescent shape; and wherein the prosthesis does not interfere with radiographic imaging.

It is another object of the invention to provide a prosthesis for mastopexy procedures and breast reconstruction surgery comprising two or more superimposed, chemically bonded layers of collagenous material which, when implanted into a mammalian patient, undergoes controlled biodegradation occurring with adequate living cell replacement such that the original implanted prosthesis is remodeled by the patient's living cells. The collagenous material may be a processed tissue material derived from intestine, dermis, fascia lata, pericardium or dura mater; however, when derived from the submucosa of small intestine, the collagenous material offers thinness, strength, and is substantially a purified collagen material that is easily handleable for preparing multi-layered prostheses.

Performance of mastopexy and breast reconstruction devices are improved when purified collagenous materials are employed, thus substantially purified collagen is a preferred material for these devices. An advantage of a mastopexy or breast reconstruction prosthesis made of collagenous material is that the prosthesis does not interfere with radiographic imaging, such as in mammography techniques to image breast tissue. These prostheses are prepared to have an elongated arcuate or crescent shape and are provided with a mesh, an arrangement of a plurality of slits running in parallel across the prostheses in the lengthwise direction, which slits are also in staggered arrangement so as to allow the prostheses to be stretched in the direction perpendicular to the slit direction in order to open the slits of the mesh but not in the direction of the slits so as to preserve the strength of the prosthesis in that direction.

### DESCRIPTION OF THE FIGURES

Figure 1 shows the mastopexy device of the invention.
Figure 2 shows the mastopexy device of the invention with closed mesh slits.
Figure 3 shows the mastopexy device of the invention with closed mesh slits in the central region of the device.
Figure 4 shows the mastopexy device of the invention with closed mesh slits at the central, apical region of the device.
Figure 5 shows the mastopexy device of the invention with closed mesh slits throughout the device except at the border of the device.
Figure 6 shows the mastopexy device of the invention with closed mesh slits throughout the device except at the border and base region of the device.
Figure 7 shows the breast reconstruction prosthesis of the invention.
Figure 8 shows the breast reconstruction prosthesis of the invention with closed mesh slits throughout the device.
Figure 9 shows the breast reconstruction prosthesis of the invention with closed mesh slits throughout the device except at the border of the device.

### DETAILED DESCRIPTION OF THE INVENTION

This invention is directed to tissue engineered prostheses made from processed tissue matrices derived from native tissues that are biocompatible with the patient or host in which they are implanted. When implanted into a mammalian host, these prostheses can serve as a functioning repair, augmentation, or replacement body part or tissue structure.

The prostheses of the invention are bioremodelable and will undergo controlled biodegradation occurring concomitantly with remodeling and replacement by the host's cells. The prosthesis of this invention, when used as a replacement tissue, thus has dual properties: First, it functions as a substitute body part, and second, while still functioning as a substitute body part, it functions as a remodeling template for the ingrowth of host cells. In order to do this, the prosthetic material of this invention is a processed tissue matrix developed from mammalian derived collagenous tissue that is able to be bonded to itself or another processed tissue matrix to form a prosthesis for grafting to a patient.

The invention is directed toward methods for making tissue engineered prostheses from cleaned tissue material where the methods do not require adhesives, sutures, or staples to bond the layers together while maintaining the bioremodelability of the prostheses. The terms "processed tissue matrix" and "processed tissue material" mean native, normally cellular tissue that has been procured from an animal source, preferably a mammal, and mechanically cleaned of attendant tissues and chemically cleaned of cells, cellular debris, and rendered substantially free of non-collagenous extracellular matrix components. The processed tissue matrix, while substantially free of non-collagenous components, maintains much of its native matrix structure, strength, and shape. Preferred compositions for preparing the bioengineered grafts of the invention are animal tissues comprising collagen collagenous tissue sources including, but not limited to: intestine, dermis, fascia lata, pericardium, dura mater, and other flat or planar structured tissues that comprise a collagenous tissue matrix. The structure of these tissue matrices makes them able to be easily cleaned, manipulated, and assembled in a way to prepare the bioengineered prostheses of the invention. Other suitable sources with the same flat structure and matrix composition may be identified, procured and processed by the skilled artisan in other animal sources in accordance with the invention.

A more preferred composition for preparing the bioengineered grafts of the invention is an intestinal collagen layer derived from the tunica submucosa of small intestine. The submucosa of small intestine is a preferred source as it yields a substantially pure collagenous material that is thin, strong, and easily handleable for preparing multi-layered prostheses of the invention. Suitable sources for small intestine are mammalian organisms such as human, cow, pig, sheep, dog, goat, or horse while small intestine of pig is the preferred source.

The most preferred composition for preparing the prosthesis of the invention is a processed intestinal collagen layer derived the tunica submucosa of porcine small intestine. To obtain the processed ICL, the small intestine of a pig is harvested and attendant mesenteric tissues are grossly dissected from the intestine. The tunica submucosa is preferably separated, or delaminated, from the other layers of the small intestine by mechanically squeezing the raw intestinal material between opposing rollers to remove the muscular layers (tunica muscularis) and the mucosa (tunica mucosa). The tunica submucosa of the small intestine is harder and stiffer than the surrounding tissue, and the rollers squeeze the softer components from the submucosa, resulting in a chemically cleaned tissue matrix. In the examples that follow, the porcine small intestine was mechanically cleaned using a Bitterling gut cleaning machine and then chemically cleaned to yield a processed tissue matrix. This mechanically and chemically cleaned intestinal collagen layer is herein referred to as "ICL". ICL is the most preferred processed tissue matrix for preparing the prostheses of the invention.

ICL is essentially acellular telopeptide Type I collagen, about 93% by weight dry, with less than about 5% dry weight glycoproteins, glycosaminoglycans, proteoglycans, lipids, non-collagenous proteins and nucleic acids such as DNA and RNA and is substantially free of cells and cellular debris. The processed ICL retains much of its matrix structure and its strength. Importantly, the biocompatability and bioremodelability of the tissue matrix is preserved in part by the cleaning process as it is free of bound detergent residues that would adversely affect the bioremodelability of the collagen. Additionally, the collagen molecules have retained their telopeptide regions as the tissue has not undergone treatment with enzymes during the cleaning process. Prostheses made from ICL also retain these characteristics.

The processed tissue matrix is used as a single layer graft prosthesis or is formed into a multi-layered, bonded prosthesis. The processed tissue matrix layers of the multilayered, bonded prosthetic device of the invention may be from the same collagen material, such as two or more layers of ICL, or from different collagen materials, such as one or more layers of ICL and one or more layers of fascia lata.

The processed tissue matrices may be treated or modified, either physically or chemically, prior to or after fabrication of a multi-layered, bonded graft prosthesis. Physical modifications such as shaping, conditioning by stretching and relaxing, or perforating the cleaned tissue matrices may be performed as well as chemical modifications such as binding growth factors, selected extracellular matrix components, genetic material, and other agents that would affect bioremodeling and repair of the body part being treated, repaired, or replaced.

A preferred physical modification is the addition of perforations, fenestrations or laser drilled holes. The tissue repair fabric can be laser drilled to create micron sized pores through the completed prosthesis for aid in cell ingrowth using an excimer laser (e.g. at KrF or ArF wavelengths). The pore size can vary from 10 to 500 microns, but is preferably from about 15 to 50 microns and spacing can vary, but about 500 microns on center is preferred. The tissue repair fabric can be laser drilled at any time during the process to make the prosthesis, but is preferably done before decontamination or sterilization. For some indications it is preferred that the perforations or laser-drilled holes communicate through all layers of the prosthesis to aid in cell passage or fluid drainage. For other indications, it is preferred that they do not pass all the away across the layers so that the holes provide cell access to the interior of a multiplayer construct or to aid in neovascularization of the construct.

A preferred chemical modification is chemical crosslinking using a crosslinking agent. While chemical crosslinking is used to bond multiple layers of processed tissue matrix together, the degree of chemical crosslinking may be varied to modulate rates of bioremodeling, that is the rates at which a prosthesis is both resorbed and replaced by host cells and tissue. In other words, the higher degree of crosslinking that is imparted to the prostheses of the invention, the slower the rate of bioremodeling the prostheses will undergo; the lower degree of crosslinking, the faster the rate of bioremodeling. Surgical indications dictate the extent of bioremodeling required by the prosthesis. For example, when a single layer construct is used as a wound dressing, no chemical crosslinking is desired. A surgical repair patch is a multilayer construct that has a low degree of crosslinking so that the prosthesis will bioremodel at a fast rate. A bladder sling to support a hypermobile bladder to prevent urinary incontinence is a multilayer construct that has a high degree of crosslinking so that the prosthesis is not bioremodeled, that is, it persists in substantially the same conformation in which it was implanted.

As ICL is the preferred starting material for the production of the bioengineered graft prostheses of the invention, the methods described below are the preferred methods for producing bioengineered graft prostheses comprising ICL.

In the most preferred embodiment, the tunica submucosa of porcine small intestine is used as a starting material for the bioengineered graft prosthesis of the invention. The small intestine of a pig is harvested, its attendant tissues removed and then mechanically cleaned using a gut cleaning machine which forcibly removes the fat, muscle and mucosal layers from the tunica submucosa using a combination of mechanical action and washing using water. The mechanical action can be described as a series of rollers that compress and strip away the successive layers from the tunica submucosa when the intact intestine is run between them. The tunica submucosa of the small intestine is comparatively harder and stiffer than the surrounding tissue, and the rollers squeeze the softer components from the submucosa. The result of the machine cleaning was such that the submucosal layer of the intestine solely remained, a mechanically cleaned intestine.

After mechanical cleaning, a chemical cleaning treatment is employed to remove cell and matrix components from the mechanically cleaned intestine, preferably performed under aseptic conditions at room temperature. The mechanically cleaned intestine is cut lengthwise down the lumen and then cut into sections approximately 15 cm in length. Material is weighed and placed into containers at a ratio of about 100:1 v/v of solution to intestinal material. In the most preferred chemical cleaning treatment, such as the method disclosed in US Patent No. 5,993,844 to Abraham, the disclosure of which is incorporated herein, the collagenous tissue is contacted with an effective amount of chelating agent, such as ethylenediaminetetraacetic tetrasodium salt (EDTA) under alkaline conditions, preferably by addition of sodium hydroxide (NaOH); followed by contact with an effective amount of acid where the acid contains a salt, preferably hydrochloric acid (HC1) containing sodium chloride (NaC1); followed by contact with an effective amount of buffered salt solution such as 1 M sodium chloride (NaC1)/10 mM phosphate buffered saline (PBS); finally followed by a rinse step using water. Each treatment step is preferably carried out using a rotating or shaking platform to enhance the actions of the chemical and rinse solutions. The result of the cleaning processes is ICL, a mechanically and chemically cleaned processed tissue matrix derived from the tunica submucosa of small intestine. After rinsing, the ICL is then removed from each container and the ICL is gently compressed of excess water. At this point, the ICL may be stored frozen at -80°C, at 4 °C in sterile phosphate buffer, or dry until use in fabrication of a prosthesis. If stored dry, the ICL sheets are flattened on a surface such as a flat plate, preferably a porous plate or membrane, such as a polycarbonate membrane, and any lymphatic tags from the abluminal side of the material are removed using a scalpel, and the ICL sheets are allowed to dry in a laminar flow hood at ambient room temperature and humidity.

The ICL is a planar sheet structure that can be used to fabricate various types of constructs to be used as prostheses with the shape of the prostheses ultimately depending on their intended use. To form prostheses of the invention, the sheets are fabricated using a method that continues to preserve the biocompatibility and bioremodelability of the processed matrix material but also is able to maintain its strength and structural characteristics for its performance as a replacement tissue. The processed tissue matrix derived from tissue retains the structural integrity of the native tissue matrix, that is, the collagenous matrix structure of the original tissue remains substantially intact and maintains physical properties so that it will exhibit many intrinsic and functional properties when implanted. Sheets of processed tissue matrix are layered to contact another sheet. The area of contact is a bonding region where layers contact, whether the layers be directly superimposed on each other, or partially in contact or overlapping for the formation of more complex structures. In completed constructs, the bonding region must be able to withstand suturing and stretching while being handled in the clinic, during implantation and during the initial healing phase while functioning as a replacement body part. The bonding region must also maintain sufficient strength until the patient's cells populate and subsequently bioremodel the prosthesis to form a new tissue.

The invention is also directed at methods for treating a patient using a biocompatible prosthesis. The prostheses of the invention are biocompatible. Biocompatibility testing has been performed on prostheses made from ICL in accordance with both Tripartite and ISO-10993 guidance for biological evaluation of medical devices. Biocompatible means that the prostheses of the invention are non-cytotoxic, hemocompatible, non-pyrogenic, endotoxin-free, non-genotoxic, non-antigenic, and do not elicit a dermal sensitization response, do not elicit a primary skin irritation response, do not case acute systemic toxicity, and do not elicit subchronic toxicity.

Test articles of the prostheses of the invention showed no biological reactivity (Grade 0) or cytotoxicity observed in the L929 cells following the exposure period test article when using the test entitled "L929 Agar Overlay Test for Cytotoxicity In Vitro." The observed cellular response to the positive control article (Grade 3) and the negative control article (Grade 0) confirmed the validity of the test system. Testing and evaluations were conducted according to USP guidelines. Prostheses of the invention are considered non-cytotoxic and meet the requirements of the L929 Agar Overlay Test for Cytotoxicity In Vitro.

Hemocompatibility (in vitro hemolysis, using the in vitro, modified ASTM - extraction method test) testing of prostheses of the invention was conducted according to the modified ASTM extraction method. Under the conditions of the study, the mean hemolytic index for the device extract was 0% while positive and negative controls performed as anticipated. The results of the study indicate the prostheses of the invention are non-hemolytic and hemocompatible.

Prostheses of the invention were subjected to pyrogenicity testing following the current USP protocol for pyrogen testing in rabbits. Under conditions of the study, the total rise of rabbit temperatures during the observation period was within acceptable USP limits. Results confirmed that the prostheses of the invention are non-pyrogenic. The prostheses of the invention are endotoxin free, preferably to a level ≤0.06 EU/ml (per cm² of product). Endotoxin refers to a particular pyrogen that is part of the cell wall of gram-negative bacteria, which is shed by the bacteria and contaminates materials.

Prostheses of the invention do not elicit a dermal sensitization response. There are no reports in the literature that would indicate that the chemicals used to clean the porcine intestinal collagen elicit a sensitization response, or would modify the collagen to elicit a response. The results of sensitization testing on prostheses of the invention formed from chemically cleaned ICL indicate that the prostheses do not elicit a sensitization response.

Prostheses of the invention do no elicit a primary skin irritation response. The results of irritation testing on the chemically cleaned ICL indicate that prostheses of the invention formed from chemically cleaned ICL do not elicit a primary skin irritation response.

Acute systemic toxicity and intracutaneous toxicity testing was performed on chemically cleaned ICL used to prepare prostheses of the invention, the results of which demonstrated a lack of toxicity among the prostheses tested. Additionally, in animal implant studies there was no evidence that chemically cleaned porcine intestinal collagen caused acute systemic toxicity.

Subchronic toxicity testing of the prostheses of the invention containing porcine intestinal collagen confirmed lack of device subchronic toxicity.

There are no reports in the literature that would indicate that the chemicals used to clean the porcine intestinal collagen would affect the potential for genotoxicity, or would modify the collagen to elicit a response. Genotoxicity testing of the prostheses of the invention containing porcine intestinal collagen confirmed lack of device genotoxicity.

The purpose of the chemical cleaning process for the porcine intestinal collagen used to prepare prostheses of the invention is to minimize antigenicity by removing cells and cell remnants. Prostheses of the invention containing porcine intestinal collagen confirmed lack of device antigenicity, as confirmed by implant studies conducted with the chemically cleaned porcine intestinal collagen.

The ICL constructs of the invention are preferably rendered virally inactivated. In the manufacturing process, the efficacy of two chemical cleaning procedures, the NaOH/EDTA alkaline chelating solution (pH 11-12) and the HCL/NaCl acidic salt solution (pH 0-1), to inactivate four relevant and model viruses was tested. The model viruses were chosen based on the source porcine material, and to represent a wide range of physicochemical properties (DNA, RNA, enveloped and non-enveloped viruses). The viruses included pseudorabies virus, bovine viral diarrhea virus, reovirus-3 and porcine parvovirus. The studies were conducted based on FDA and ICH guidance documents, including: CBER/FDA "Points to Consider in the Characterization of Cell Lines Used to Produce Biologicals (1993)"; ICH "Note for Guidance on Quality of Biotechnological Products: Viral Safety Evaluation of Biotechnology Products Derived from Cell Lines of Human or Animal Origin" (CPMP/ICH/295/95); and, CPMP Biotechnology Working Party "Note for Guidance on Virus Validation Studies: The Design, Contribution and Interpretation of Studies Validating the Inactivation and Removal of Viruses" (CPMPBWP/268/95). The results of the study demonstrate that the cumulative viral inactivation of the two chemical cleaning steps is a clearance of greater than 10⁶ for all four model viruses. The data indicate that the chemical cleaning procedures are a robust and effective process that maintains the potential for inactivation of a large variety of viral agents.

In a preferred embodiment, the prosthetic device of the invention is a single layer of processed tissue matrix, preferably ICL that has been mechanically and chemically cleaned, that is biocompatible and bioremodelable for use as a surgical graft prosthesis, or more preferably, as a wound dressing. A preferred modification to the single layer construct is the addition of perforations or fenestrations that communicate between both sides of the construct. To make a single layer ICL construct, ICL is spread mucosal side down onto a smooth polycarbonate sheet; ensuring removal of creases, air bubbles and visual lymphatic tags. Spreading of the ICL over the polycarbonate sheet is performed to optimize the dimensions. Material is adequately dried over its entire surface. Material is fenestrated and then cut to size and packaged and finally sterilized per sterilization specifications.

In another preferred embodiment, the prosthetic device of this invention has two or more superimposed collagen layers that are bonded together. As used herein, "bonded collagen layers" means composed of two or more layers of the same or different collagen material treated in a manner such that the layers are superimposed on each other and are sufficiently held together by self-lamination and chemical crosslinking.

In a most preferred embodiment, surgical device is a flat sheet construct consisting of five layers of ICL, bonded and crosslinked with 1 mM with 1-ethyl-3-(3-dimethylaminopropyl) carbodiimide hydrochloride (EDC) in water. To form this construct, a first sheet of ICL is spread mucosal side down onto a smooth polycarbonate sheet; ensuring removal of creases, air bubbles and visual lymphatic tags. Spreading of the ICL is done to optimize dimensions. Three sheets of ICL (mucosal side down) are layered on top of the first, ensuring removal of creases, air bubbles and visual lymphatic tags when each sheet is layered. The fifth sheet should be layered with the mucosal side facing up, ensuring removal of creases and air bubbles. Visual lymphatic tags are removed prior to layering of this fifth sheet. The layers are dried together for 24 ± 8 hours. The layers are now dried together and then are crosslinked in 1 mM EDC in water for 18 ±2 hours in 500 mL of crosslinking solution per 30 cm five layer sheet. Each product is rinsed with sterile water and is then cut to final size specifications while hydrated.

In another more preferred embodiment, the prosthetic device is a surgical sling that is intended for implantation to reinforce and support soft tissues where weakness exists including but not limited to the following procedures: pubourethral support, prolapse repair (urethral, vaginal, rectal and colon), reconstruction of the pelvic floor, bladder support, sacrocolposuspension, reconstructive procedures and tissue repair. In another most preferred embodiment, the prosthetic device is a surgical sling comprised of three to five layers of bonded, crosslinked ICL. To fabricate a five layer device, ICL is spread mucosal side down onto a smooth polycarbonate sheet; ensuring removal of creases, air bubbles and visual lymphatic tags. Spreading of the ICL is done to optimize dimensions. A second, third, and fourth sheets of ICL (mucosal side down) are layered on top of the first, ensuring removal of creases, air bubbles and visual lymphatic tags when each sheet is layered. The fifth sheet is layered with the mucosal side facing up, ensuring removal of creases and air bubbles. Visual lymphatic tags should be removed prior to layering of this fifth sheet. (A three layer construct is made by a first sheet of ICL spread mucosal side down onto a smooth polycarbonate sheet; ensuring removal of creases, air bubbles and visual lymphatic tags; a second sheet of ICL (mucosal side down) layered on top of the first, and a third sheet layered on top of the second sheet with the mucosal side facing up.) The layers are dried for 24 ± 8 hours and once dry, are crosslinked in 10 mM EDC in 90% acetone for 18 ±2 hours in 500 mL of crosslinking solution per 30 cm five layer sheet. Each bonded, crosslinked construct is rinsed with sterile water and is cut to final size specifications while hydrated. By providing pubourethral support, the sling may be used for the treatment of urinary incontinence resulting from urethral hypermobility or intrinsic sphincter deficiency. The surgical sling consists of a five-layer laminated sheet of porcine intestinal collagen, about 0.20 mm to about 0.25 mm in thickness. The device is cross-linked with 1-ethyl-3-(3-dimethylaminopropyl) carbodiimide hydrochloride (EDC). The device consists primarily of Type I porcine collagen (about >95%) in its native form, with less than about 0.7% lipids and undetectable levels of glycosaminoglycans (about <0.6%) and DNA (about <0.1 Ng/µl). The porcine intestinal collagen is free of cells and cell remnants. The denaturation temperature (DSC) of the prosthesis is greater than about 63°C; it's tensile strength is greater than about 15N; it's suture retention strength is greater than about 2N using a 2-0 braided silk suture; and the final endotoxin level its ≤0.06 EU/ml (per cm² of product). While the bioremodelable aspects of the sling can be varied and leveraged, the sling prosthesis of the invention is not a replacement body part, but and organ support device implanted as an assisting structure, it is preferred that the ICL layers of the sling be more highly crosslinked to reduce the bioremodelability of the sling. The sling prosthesis is highly biocompatible, flexible, collagenous structure that, when implanted, maintains requisite structural support and strength while functioning as an organ support device.

A preferred embodiment of the invention is directed to flat sheet prostheses, and methods for making and using flat sheet prostheses, comprising of two or more layers of ICL bonded and crosslinked for use as an implantable biomaterial capable of being bioremodeled by a patient's cells. Due to the flat sheet structure of ICL, the prosthesis is easily fabricated to comprise any number of layers, preferably between 2 and 10 layers, more preferably between 2 and 6 layers, with the number of layers depending on the strength and bulk necessary for the final intended use of the construct. The ICL has structural matrix fibers that run in the same general direction. When layered, the layer orientations may be varied to leverage the general tissue fiber orientations in the processed tissue layers. The sheets may be layered so their fiber orientations are in parallel or at different angles. Layers may also be superimposed to form a construct with continuous layers across the area of the prosthesis. Alternatively, as the ultimate size of a superimposed arrangement is limited by the circumference of the intestine, the layers may be staggered, in collage arrangement to form a sheet construct with a surface area larger than the dimensions of the starting material but without continuous layers across the area of the prosthesis. Complex features may be introduced such as a conduit or network of conduit or channels running between the layers or traversing the layers, for example.

In the fabrication of a multilayer construct comprising ICL, an aseptic environment and sterile tools are preferably employed to maintain sterility of the construct when starting with sterile ICL material. To form a multilayer construct of ICL, a first sterile rigid support member, such as a rigid sheet of polycarbonate, is laid down in the sterile field of a laminar flow cabinet. If the ICL sheets are still not in a hydrated state from the mechanical and chemical cleaning processes, they are hydrated in aqueous solution, such as water or phosphate buffered saline. ICL sheets are blotted with sterile absorbent cloths to absorb excess water from the material. If not yet done, the ICL material is trimmed of any lymphatic tags on the serosal surface, from the abluminal side. A first sheet of trimmed ICL is laid on the polycarbonate sheet and is manually smoothed to the polycarbonate sheet to remove any air bubbles, folds, and creases. A second sheet of trimmed ICL is laid on the top of the first sheet, again manually removing any air bubbles, folds, and creases. This is repeated until the desired number of layers for a specific application is obtained, preferably between 2 and 10 layers.

The ICL has a sidedness quality from its native tubular state: an inner mucosal surface that faced the intestinal lumen in the native state and an opposite outer serosal surface that faced the ablumen. It has been found that these surfaces have characteristics that can affect post-operative performance of the prosthesis but can be leveraged for enhanced device performance. Currently with the use of synthetic devices, adhesion formation may necessitate the need for re-operation to release the adhesions from the surrounding tissue. In the formation of a pericardial patch or hernia repair prosthesis having two layers of ICL, it is preferred that the bonding region of the two layers is between the serosal surfaces as the mucosal surfaces have demonstrated to have an ability to resist postoperative adhesion formation after implantation. In other embodiments, it is preferred that one surface of the ICL patch prosthesis be non-adhesive and the other surface have an affinity for adhering to host tissue. In this case, the prosthesis will have one surface mucosal and the other surface serosal. In still another embodiment, it is preferred that the opposing surfaces be able to create adhesions to grow together tissues that contact it on either side, thus the prosthesis will have serosal surfaces on both sides of the construct. Because only the two outer sheets potentially contact other body structures when implanted, the orientation of the internal layers, if the construct is comprised of more than two, is of lesser importance as they will likely not contribute to post-operative adhesion formation.

After layering the desired number of ICL sheets, they are then bonded by dehydrating them together at their bonding regions, that is, where the sheets are in contact. While not wishing to be bound by theory, dehydration collagen fibers of the ICL layers together when water is removed from between the fibers of the ICL matrix. The layers may be dehydrated either open-faced on the first support member or, between the first support member and a second support member, such as a second sheet of polycarbonate, placed before drying over the top layer of ICL and fastened to the first support member to keep all the layers in flat planar arrangement together with or without a small amount of pressure. To facilitate dehydration, the support member may be porous to allow air and moisture to pass through to the dehydrating layers. The layers may be dried in air, in a vacuum, or by chemical means such as by acetone or an alcohol such as ethyl alcohol or isopropyl alcohol. Dehydration may be done to room humidity, between about 10% Rh to about 20% Rh, or less; or about 10% to about 20% w/w moisture, or less. Dehydration may be easily performed by angling the frame holding the polycarbonate sheet and the ICL layers up to face the oncoming airflow of the laminar flow cabinet for at least about 1 hour up to 24 hours at ambient room temperature, approximately 20 °C, and at room humidity.

While it is not necessary, in the preferred embodiment, the dehydrated layers are rehydrated before crosslinking. The dehydrated layers of ICL are peeled off the porous support member together and are rehydrated in an aqueous rehydration agent, preferably water, by transferring them to a container containing aqueous rehydration agent for at least about 10 to about 15 minutes at a temperature between about 4 °C to about 20 °C to rehydrate the layers without separating or delaminating them.

The dehydrated, or dehydrated and rehydrated, bonded layers are then crosslinked together at the bonding region by contacting the layered ICL with a crosslinking agent, preferably a chemical crosslinking agent that preserves the bioremodelability of the ICL material. As mentioned above, the dehydration brings the collagen fibers in the matrices of adjacent ICL layers together and crosslinking those layers together forms chemical bonds between the components to bond the layers together. Crosslinking the bonded prosthetic device also provides strength and durability to the device to improve handling properties and to control rates of bioremodeling. As a general rule, a greater degree of crosslinking results in a longer time for the material to bioremodel. Various types of crosslinking agents are known in the art and can be used such as carbodiimides, genipin, transglutaminase, ribose and other sugars, nordihydroguaiaretic acid (NDGA), oxidative agents, ultraviolet (UV) light and dehydrothermal (DHT) methods.. Aldehydes, such as glutaraldehyde, are also employed as a reagent in crosslinking; however, data from studies using glutaraldehyde as the crosslinking agent are hard to interpret since glutaraldehyde treatment is also known to leave behind cytotoxic residues. It is, therefore, possible that the reduced antigenicity associated with glutaraldehyde crosslinking is due to non-specific cytotoxicity rather than a specific effect on antigenic determinants. Glutaraldehyde crosslinking is one way to increase durability and reduce antigenicity of collagenous materials as compared to those that are noncrosslinked but glutaraldehyde crosslinking collagen materials significantly limits the body's ability to remodel the prosthesis as the cytoxic residues reduce cell compatibility with the collagen. It has been shown that glutaraldehyde treated valve leaflets become calcified when implanted in vivo. Therefore, aldehyde crosslinking is not preferred. An ideal crosslinking is one that bonds the layers of processed tissue matrix together while preserving handleability and bioremodelability.

A preferred crosslinking agent is 1-ethyl-3-(3-dimethylaminopropyl) carbodiimide hydrochloride (EDC). In an another preferred method, sulfo-N-hydroxysuccinimide is added to the EDC crosslinking agent as described by Staros, J.V., Biochem. 21, 3950-3955, 1982. In the most preferred method, EDC is solubilized in water at a concentration preferably between about 0.1 mM to about 100 mM, more preferably between about 1.0 mM to about 10 mM, most preferably at about 1.0 mM. Besides water, phosphate buffered saline or (2-[N-morpholino]ethanesulfonic acid) (MES) buffer may be used to dissolve the EDC. Other agents may be added to the solution, such as acetone or an alcohol, up to 99% v/v in water, typically 50%, to make crosslinking more uniform and efficient. These agents remove water from the layers to bring the matrix fibers together to promote crosslinking between those fibers. The ratio of these agents to water in the crosslinking agent can be used to regulate crosslinking. EDC crosslinking solution is prepared immediately before use as EDC will lose its activity over time. To contact the crosslinking agent to the ICL, the hydrated, bonded ICL layers are transferred to a container such as a shallow pan and the crosslinking agent gently decanted to the pan ensuring that the ICL layers are both covered and free-floating and that no air bubbles are present under or within the layers of ICL constructs. The container is covered and the layers of ICL are allowed to crosslink for between about 4 to about 24 hours, more preferably between 8 to about 16 hours at a temperature between about 4 °C to about 20 °C. Crosslinking can be regulated with temperature: At lower temperatures, crosslinking is more effective as the reaction is slowed; at higher temperatures, crosslinking is less effective as the EDC is less stable.

After crosslinking, the crosslinking agent is decanted and disposed of and the constructs are rinsed in the pan by contacting them with a rinse agent to remove residual crosslinking agent. A preferred rinse agent is water or other aqueous solution. Preferably, sufficient rinsing is achieved by contacting the chemically bonded construct three times with equal volumes of sterile water for about five minutes for each rinse. Using a scalpel and ruler, constructs are trimmed to the desired size; a usable size is about 6 inches square (approx. 15.2 cm x 15.2 cm) but any size may be prepared and used for grafting to a patient. Constructs may be also cut and trimmed to a desired shape and size using methods known in the art for cutting collagenous biomaterials.

Constructs are then terminally sterilized using means known in the art of medical device sterilization. A preferred method for sterilization is by contacting the constructs with sterile 0.1% peracetic acid (PA) treatment neutralized with a sufficient amount of 10 N sodium hydroxide (NaOH), according to US Patent No. 5,460,962, the disclosure of which is incorporated herein. Decontamination is performed in a container on a shaker platform, such as 1 L Nalge containers, for about 18 ± 2 hours. Constructs are then rinsed by contacting them with three volumes of sterile water for 10 minutes each rinse. In a more preferred method, ICL constructs are sterilized using gamma irradiation between 25-37 kGy. Gamma irradiation significantly, but not detrimentally, decreases Young's modulus, ultimate tensile strength, and shrink temperature. The mechanical properties after gamma irradiation are still sufficient for use in a range of applications and gamma is a preferred means for sterilizing as it is widely used in the field of implantable medical devices. Dosimetry indicators are included with each sterilization run to verify that the dose is within the specified range. Constructs are packaged using a package material and design that ensures sterility during storage. A preferred packaging means is a double-layer peelable package where the principal package is a heat-sealed, blister package comprised of a polyethylene terephthalate, glycol modified (PETG) tray with a paper surfaced foil lid that is enclosed in a secondary heat sealed pouch comprised of a polyethelene/polyethyleneterephthalate (PET) laminate. Together, both the principal and secondary package and the ICL construct contained therein are sterilized using gamma radiation.

In still another preferred embodiment, after ICL is reformed into a construct for tissue repair or replacement, it may be populated with cells to form a cellular tissue construct comprising bonded layers of ICL and cultured cells. Cellular tissue constructs can be formed to mimic the organs they are to repair or replace.

Cell cultures are established from mammalian tissue sources by dissociating the tissue or by explant method. Primary cultures are established and cryopreserved in master cell banks from which portions of the bank are thawed, seeded, and subcultured to expand cell numbers. To populate an acellular ICL construct with cells, the construct is placed in a culture dish or flask and contacted by immersion in media containing suspended cells. Because collagen is a natural substance for cell adhesion, cells bind to the ICL construct and proliferate on and into the collagenous matrix of the construct.

Preferred cell types for use in this invention are derived from mesenchyme. More preferred cell types are fibroblasts, stromal cells, and other supporting connective tissue cells, or human dermal fibroblasts. Human fibroblast cell strains can be derived from a number of sources, including, but not limited to neonate male foreskin, dermis, tendon, lung, umbilical cords, cartilage, urethra, corneal stroma, oral mucosa, and intestine. The human cells may include but need not be limited to: fibroblasts, smooth muscle cells, chondrocytes and other connective tissue cells of mesenchymal origin. It is preferred, but not required, that the origin of the matrix-producing cell used in the production of a tissue construct be derived from a tissue type that it is to resemble or mimic after employing the culturing methods of the invention. For instance, a multilayer sheet construct is cultured with fibroblasts to form a living connective tissue construct; or myoblasts, for a skeletal muscle construct. More than one cell type can be used to populate an ICL construct, for example, a tubular ICL construct can be first cultured with smooth muscle cells and then the lumen of the construct populated with the first cell type is cultured with vascular endothelial cells as a second cell type to form a cellular vascular replacement device. Similarly, a urinary bladder wall patch prosthesis is prepared on multilayer ICL sheet constructs using smooth muscle cells as a first cell type and then urinary endothelial cells as a second cell type. Cell donors may vary in development and age. Cells may be derived from donor tissues of embryos, neonates, or older individuals including adults. Embryonic progenitor cells such as mesenchymal stem cells may be used in the invention and induced to differentiate to develop into the desired tissue.

Although human cells are preferred for use in the invention, the cells to be used in the method of the are not limited to cells from human sources. Cells from other mammalian species including, but not limited to, equine, canine, porcine, bovine, ovine, and murine sources may be used. In addition, cells that are genetically engineered by spontaneous, chemical,. or viral transfection may also be used in this invention. For those embodiments that incorporate more than one cell type, mixtures of normal and genetically modified or transfected cells may be used and mixtures of cells of two or more species or tissue sources may be used, or both.

Recombinant or genetically-engineered cells may be used in the production of the cell-matrix construct to create a tissue construct that acts as a drug delivery graft for a patient needing increased levels of natural cell products or treatment with a therapeutic. The cells may produce and deliver to the patient via the graft recombinant cell products, growth factors, hormones, peptides or proteins for a continuous amount of time or as needed when biologically, chemically, or thermally signaled due to the conditions present in the patient. Cells may also be genetically engineered to express proteins or different types of extracellular matrix components which are either 'normal' but expressed at high levels or modified in some way to make a graft device comprising extracellular matrix and living cells that is therapeutically advantageous for improved wound healing, or facilitated or directed neovascularization. These procedures are generally known in the art, and are described in Sambrook et al, Molecular Cloning, A Laboratory Manual, Cold Spring Harbor Press, Cold Spring Harbor, NY (1989), incorporated herein by reference. All of the above-mentioned types of cells may be used in this invention for the production of a cellular tissue construct formed from an acellular construct formed from bonded ICL layers.

The prostheses of this invention, functioning as a substitute body part, may be flat, tubular, or of complex geometry. The shape of the formed prosthesis will be decided by its intended use. Thus, when forming the bonding layers of the prosthesis of this invention, the mold or plate support member can be fashioned to accommodate the desired shape. The flat multilayer prostheses can be implanted to repair, augment, or replace diseased or damaged organs, such as abdominal wall, pericardium, hernias, and various other organs and structures including, but not limited to, bone, periosteum, perichondrium, intervertebral disc, articular cartilage, dermis, bowel, ligaments, and tendons. In addition, the flat multilayer prostheses can be used as a vascular or intra-cardiac patch, or as a replacement heart valve.

Flat sheets may also be used for organ support, for example, to support prolapsed or hypermobile organs by using the sheet as a sling for the organs, such as bladder or uterus. Tubular prostheses may be used, for example, to replace cross sections of tubular organs such as vasculature, esophagus, trachea, intestine, and fallopian tubes. These organs have a basic tubular shape with an outer surface and an inner luminal surface. In addition, flat sheets and tubular structures can be formed together to form a complex structure to replace or augment cardiac or venous valves.

The bioengineered graft prostheses of the invention may be used to repair or replace body structures that have been damaged or diseased in host tissue.

While functioning as a substitute body part or support, the prosthesis also functions as a bioremodelable matrix scaffold for the ingrowth of host cells. "Bioremodeling" is used herein to mean the production of structural collagen, vascularization, and cell repopulation by the ingrowth of host cells at a rate about equal to the rate of biodegradation, reforming and replacement of the matrix components of the implanted prosthesis by host cells and enzymes. The graft prosthesis retains its structural characteristics while it is remodeled by the host into all, or substantially all, host tissue, and as such, is functional as an analog of the tissue it repairs or replaces.

Young's Modulus (MPa) is defined as the linear proportional constant between stress and strain. The Ultimate Tensile Strength (N/mm) is a measurement of the strength across the prosthesis. Both of these properties are a function of the number of layers of ICL in the prosthesis. When used as a load bearing or support device, it should be able to withstand the rigors of physical activity during the initial healing phase and throughout remodeling.

Lamination strength of the bonding regions is measured using a peel test. Immediately following surgical implantation, it is important that the layers not delaminate under physical stresses. In animal studies, no explanted materials showed any evidence of delamination. Before implantation, the adhesion strength between two opposing layers is about 8.1 ± 2.1 N/mm for a 1 mM EDC crosslinked multilayer construct.

Shrink Temperature (°C) is an indicator of the extent of matrix crosslinking. The higher the shrink temperature, the more crosslinked the material. Non-crosslinked, gamma-irradiated ICL has a shrink temperature of about 60.5 ± 1.0. In the preferred embodiment, an EDC crosslinked prostheses will preferably have a shrink temperature between about 64.0 ± 0.2 °C to about 72.5 ± 1.1 °C for devices that are crosslinked in 1 mM EDC to about 100 mM EDC in 50% acetone, respectively.

The mechanical properties include mechanical integrity such that the prosthesis resists creep during bioremodeling, and additionally is pliable and suturable. The term "pliable" means good handling properties for ease in use in the clinic.

The term "suturable" means that the mechanical properties of the layer include suture retention which permits needles and suture materials to pass through the prosthesis material at the time of suturing of the prosthesis to sections of native tissue. During suturing, such prostheses must not tear as a result of the tensile forces applied to them by the suture, nor should they tear when the suture is knotted. Suturability of the prostheses, i.e., the ability of prostheses to resist tearing while being sutured, is related to the intrinsic mechanical strength of the prosthesis material, the thickness of the graft, the tension applied to the suture, and the rate at which the knot is pulled closed. Suture retention for a highly crosslinked flat 6 layer prosthesis crosslinked in 100 mM EDC and 50% acetone is about 6.7 ± 1.6 N. Suture retention for a 2 layer prosthesis crosslinked in 1 mM EDC in water is s about 3.7 N ± 0.5 N. The preferred lower suture retention strength is about 2N for a crosslinked flat 2 layer prosthesis as a surgeon's force in suturing is about 1.8 N.

As used herein, the term "non-creeping" means that the biomechanical properties of the prosthesis impart durability so that the prosthesis is not stretched, distended, or expanded beyond normal limits after implantation. As is described below, total stretch of the implanted prosthesis of this invention is within acceptable limits. The prosthesis of this invention acquires a resistance to stretching as a function of post-implantation cellular bioremodeling by replacement of structural collagen by host cells at a faster rate than the loss of mechanical strength of the implanted materials due from biodegradation and remodeling.

The processed tissue material of the present invention is "semi-permeable," even though it has been layered and bonded. Semi-permeability permits the ingrowth of host cells for remodeling or for deposition of agents and components that would affect bioremodelability, cell ingrowth, adhesion prevention or promotion, or blood flow. The "non-porous" quality of the prosthesis prevents the passage of fluids intended to be retained by the implantation of the prosthesis. Conversely, pores may be formed in the prosthesis if a porous or perforated quality is required for an application of the prosthesis.

The mechanical integrity of the prosthesis of this invention is also in its ability to be draped or folded, as well as the ability to cut or trim the prosthesis obtaining a clean edge without delaminating or fraying the edges of the construct.

Another preferred embodiment of the invention is a prosthesis for mastopexy procedures comprising two or more superimposed, chemically bonded layers of collagenous material which, when implanted into a mammalian patient, undergoes controlled biodegradation occurring with adequate living cell replacement such that the original implanted prosthesis is remodeled by the patient's living cells. In a more preferred embodiment, the processed tissue material derived from intestine, dermis, fascia lata, pericardium or dura mater, but most preferably it is derived from the submucosa of porcine small intestine. It should be noted that embodiments comprising processed tissue matrix derived from dermis, including cadaver dermis, may comprise a single layer of dermis as that tissue is harvested with different thicknesses and, as such, the material does not need to be layered and bonded; however, thicker tissue sources present challenges when cleaning to purify those tissue matrices. Performance of mastopexy devices are improved when purified collagenous materials are employed, thus purified collagen is a preferred material for these devices. An advantage of a mastopexy prosthesis made of collagenous material is that the prosthesis does not interfere with radiographic imaging, such as in mammography techniques to image breast tissue.

The mastopexy prosthesis of the invention is generally flat, elongate and gently arcuate and resembles a crescent with pointed, rounded, or squared ends. A semi-circle or half-moon shape may also be used as the shape for the prosthesis. The shape of the arch may be elliptical (similar to an ellipse halved lengthwise), parabolic, horseshoe, or of a generally similar arcuate shape. The mastopexy device of the invention comprises preferably between two and ten, more preferably three to five, most preferably four chemically crosslinked layers of processed tissue matrix having an arcuate or crescent shape and is between about 7 cm to about 12 cm wide and about 25 cm to about 35 cm long, more preferably about 9 cm to about 10 cm wide and about 28 cm to about 32 cm long. Therefore, the mastopexy device of the invention is a prosthesis for mastopexy, or breast lift, comprising two or more superimposed, chemically bonded layers of collagenous material which, when implanted into a mammalian patient, undergoes controlled biodegradation occurring with adequate living cell replacement such that the original implanted prosthesis is remodeled by the patient's living cells. The mastopexy device of the invention is preferably comprises between three to seven layers of processed tissue matrix, more preferably between four to six layers of processed tissue matrix, wherein it is even more preferred that in these embodiments of the invention that the processed tissue matrix is ICL and wherein the layers are chemically bonded with EDC. The degree of crosslinking is one that allows the processed tissue layers to substantially persist for about six months prior to the layers to bioremodel by infiltration of the patient's cells into the matrix layers to replace the implanted matrix with matrix produced by the patient's cells. Any of the crosslinking methods using EDC, described herein, may be used in the preparation of the mastopexy prostheses of the invention.

Physical modifications and features may also be added to the design. Preferably, the prosthesis is meshed either completely or partially in the general lengthwise axis of the prosthesis. The mesh provides openings that communicate between the opposing flat surfaces of the prosthesis to impart multiple performance benefits when the prosthesis is implanted. One benefit is that the mesh openings allow for fluid and cell transport between the outside surfaces of the prosthesis and may provide contact of tissues on either side of the implanted prosthesis where the allowance of this transport and contact provide improved integration and bioremodeling of the prosthesis. Another benefit of the mesh openings is that the surgeon may adjust the shape and contour of the prosthesis to affect the breast shape and curvature. The mesh is provided as an arrangement of slits running in parallel to each other with adjacent slits offset in staggered arrangement such that when the material is pulled at a direction perpendicular to the direction of the slits, the material will stretch and the slits will open such that the appearance of the material is a mesh. The direction of the slits run generally in the lengthwise direction of the prosthesis. The meshed prosthesis, when pulled in the direction of the slits, still retains its strength and support as it offers only a minimal amount of stretch. For the surgeon to better conform the device material to the features of a tissue expander or implant, the mesh arrangement allows for certain areas of the material to stretch and open the slits while allowing other areas to remain closed. More specifically, the surgeon contours the breast tissue by extending the prosthetic material at the peak of the curve of the prosthesis from under the breast and upward toward the nipple-areola complex to lift and support the underside of the breast. Doing this may open the slits more at the apical portion of the prosthesis more than those at the base portion. The size and arrangement of the slits are described as a "mesh ratio" that is defined as the difference of the expanded material as compared to its size before expansion. The length of the incisions determines the degree of expansion. The mesh ratio provided to the prostheses should be preferably between 1:1 and 3:1, more preferably at a ratio between 1:1 and 1.5:1. Other physical modifications that may be provided to the prosthesis are perforations, holes, or fenestrations along the major surface of the prosthesis. These physical modifications may be provided manually using a scalpel or biopsy punch or by use of a die-cutting machine, a water-jet cutting machine, laser, or other cutting means known in the art.

Another preferred design for the mastopexy prosthesis of the invention is a generally flat, elongate and arcuate shape and resembles a crescent with pointed, rounded, or squared ends where the center region of the prosthesis is meshed with slits that may be opened to contour the prosthesis and the end portions have perforations in a grid or staggered arrangement so as not to allow contouring but provide strength at the attachment site of the prosthesis to body tissues.

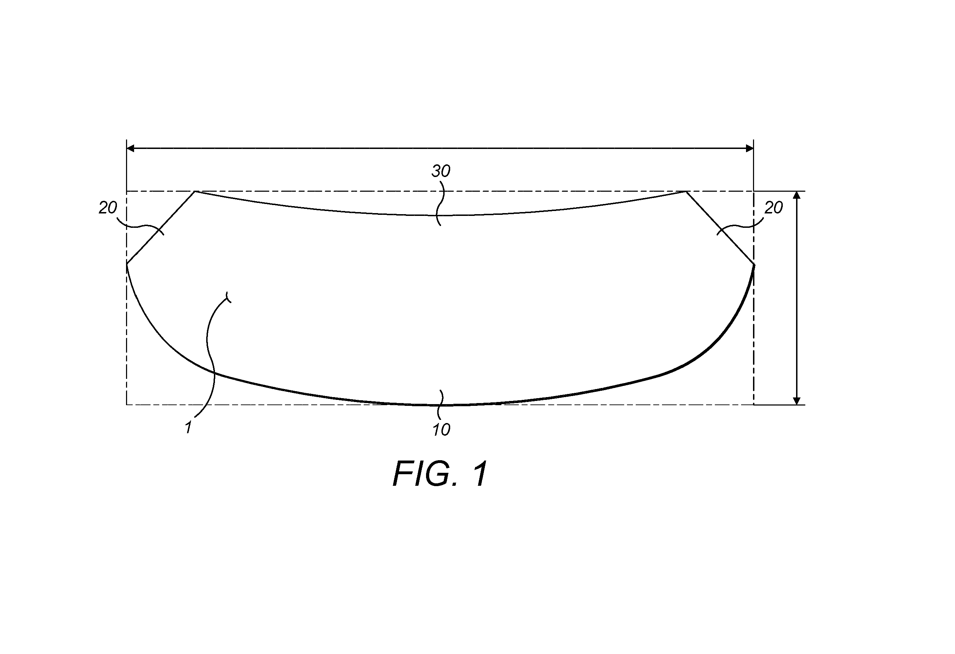

Another aspect of the invention is a method for treating a patient with a bioremodelable, collagenous mastopexy device having an elongate and arcuate shape. Figures 1 through 5 show particular mastopexy device designs of the invention.
Figure 1 shows a mastopexy device of the invention wherein mastopexy device 1 is elongate and arcuate having two squared end portions 20 with an apex 10 and base 30.

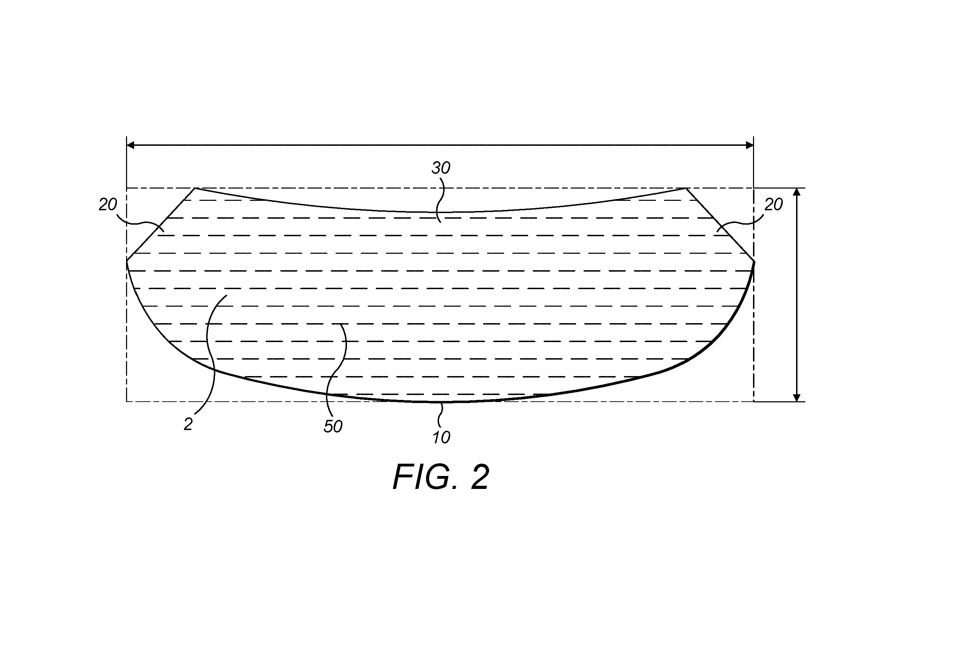

Figure 2 shows the mastopexy device of the invention with closed mesh slits wherein mastopexy device 2 is elongate and arcuate having two squared end portions 20 with an apex 10, base 30 and mesh slits 50 in a regular arrangement across the entire device.

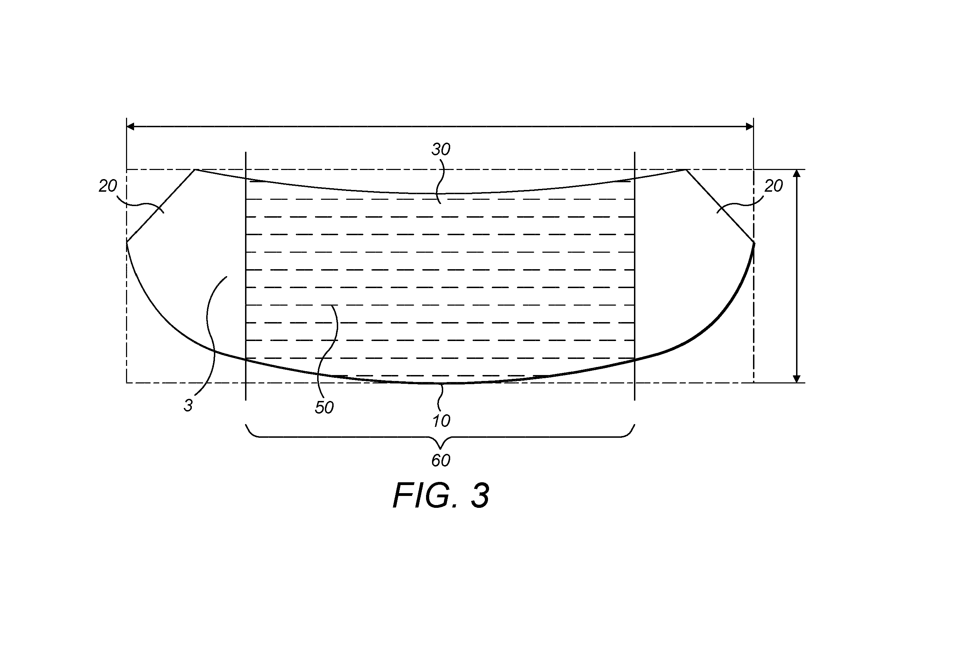

Figure 3 shows the mastopexy device 3 of the invention with closed mesh slits in the central region of the device wherein mastopexy device 3 is elongate and arcuate having two squared end portions 20 with an apex 10, base 30 and mesh slits 50 in a regular arrangement in a central portion 60 of the device.

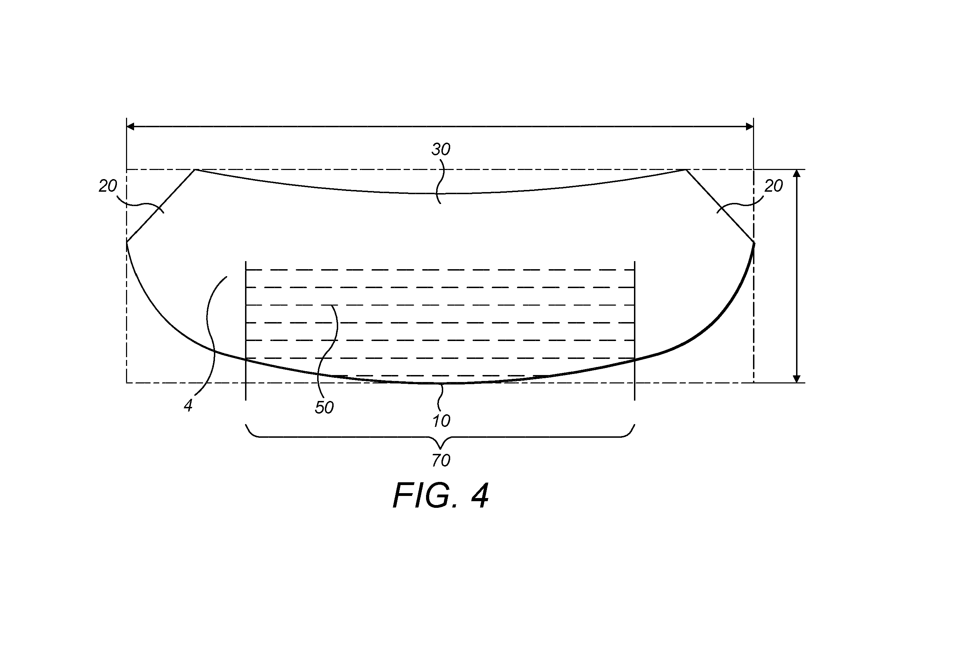

Figure 4 shows the mastopexy device 4 of the invention with closed mesh slits in the central, apical region (or apex) of the device wherein mastopexy device 3 is elongate and arcuate having two squared end portions 20 with an apex 10, base 30 and mesh slits 50 in a regular arrangement in a central, apical portion 70 of the device. End portions 20 and base 30 do not have mesh slits provided.

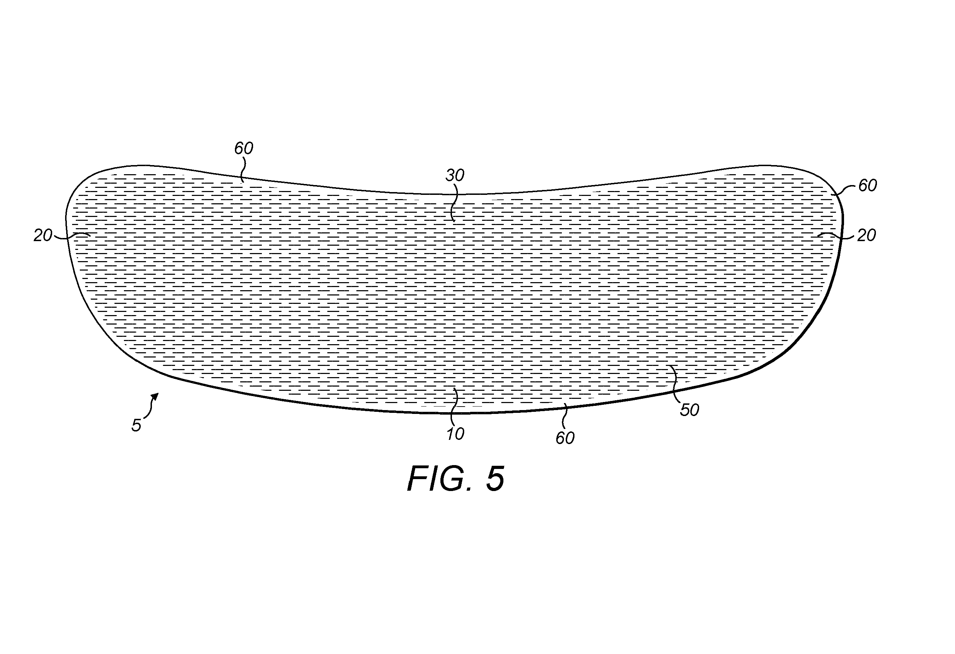

Figure 5 shows the mastopexy device 5 of the invention with closed mesh slits wherein mastopexy device 5 is elongate and arcuate having two rounded end portions 20 with an apex 10, base 30 and mesh slits 50 in a regular arrangement across the entire device except at the border 60 of the device.

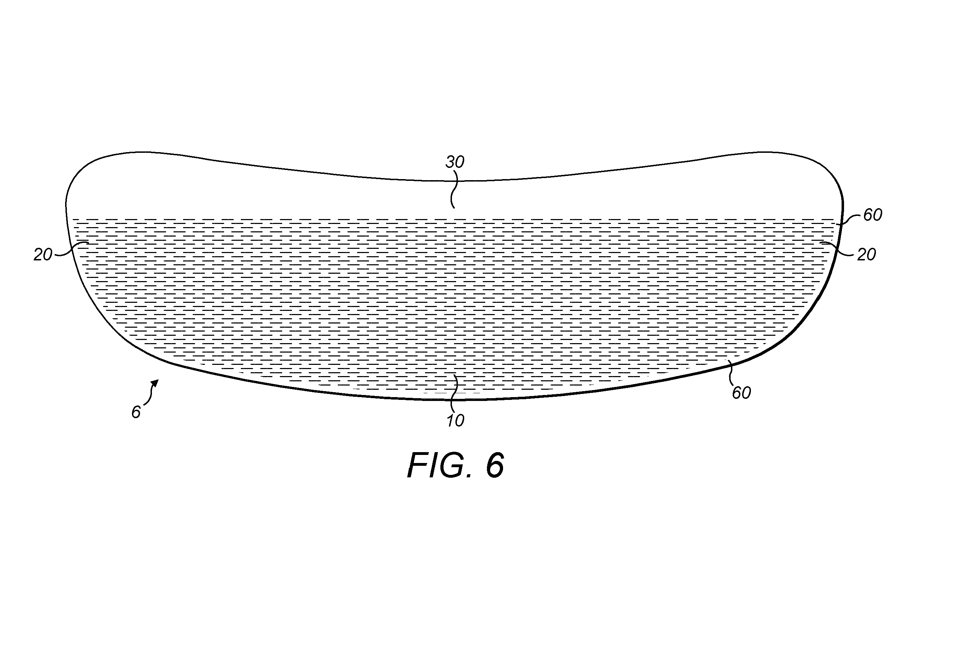

Figure 6 shows the mastopexy device 5 of the invention with closed mesh slits wherein mastopexy device 6 is elongate and arcuate having two rounded end portions 20 with an apex 10 and mesh slits 50 in a regular arrangement across the entire device except at the border 60 and base region 30 of the device. Mastopexy surgeries are performed with reduction, augmentation or neither reduction or augmentation. All three surgical techniques of the invention involve lift, support, and shaping of the breast using the mastopexy device of the present invention. Thus, the present invention includes a method for treating a patient with a mastopexy device to lift, support and shape the breast with a bioremodelable collagenous mastopexy device that allows for mammographical imaging of the breast tissue.

More specifically, the method of the present invention include methods for mastopexy treatment using the prosthesis of the invention in lift procedures with or without either breast reduction or breast augmentation procedures. Therefore, the methods of the invention include: a method for mastopexy treatment with reduction in breast volume, comprising:
1. Marking four points on the breast around the areola to determine the amount of skin necessary for both the external skin lining of the new breast and the excess skin in the periareaolar region for the dermal flap to be used for the internal skin lining
2. De-epithelializing the flap to retain the central pedicle
3. Displace the breast subcutaneous down to the level of the pectoral fascia
4. Dissecting the skin on the bias in the upper hemisphere in order to progressively increase the thickness of the subcutaneous fat tissue close to the skin and
5. Resecting a central wedge of tissue and shortening the upper hemisphere ray
6. Dissecting the skin from the parenchymal tissue in the lower hemisphere of the breast
7. Optionally resecting a second central wedge of tissue in the lower hemisphere
8. Applying a mastopexy prosthesis over the dermal flap in the lower hemisphere to sling the underside of the breast
9. Suturing the mastopexy prosthesis to the pectoralis fascia to promote elevation and shape of the mammary cone
10. Suturing closed the external skin lining while fixing the areolar skin to the external skin lining
11. Dressing the breast in a supportive way that allows drainage of exudates.

The method of the invention also includes a mastopexy treatment to lift breast tissue comprising
1. Marking four points on the breast around the areola to determine the amount of skin necessary for both the external skin lining of the new breast and the excess skin in the periareaolar region for the dermal flap to be used for the internal skin lining
2. De-epithelializing the flap to retain the central pedicle
3. Displace the breast subcutaneous down to the level of the pectoral fascia
4. Dissecting the skin on the bias in the upper hemisphere in order to progressively increase the thickness of the subcutaneous fat tissue close to the skin and
5. Dissecting the skin from the parenchymal tissue in the lower hemisphere of the breast
6. Applying a mastopexy prosthesis over the dermal flap in the lower hemisphere to sling the underside of the breast
7. Suturing the mastopexy prosthesis to the pectoralis fascia to promote elevation and shape of the mammary cone
8. Suturing closed the external skin lining while fixing the areolar skin to the external skin lining
9. Dressing the breast in a supportive way that allows drainage of exudates.

The method of the invention further includes a method for mastopexy treatment with augmentation (performed in either one or two operations) where the method comprises:
1. Inserting a breast implant either under the muscle in a submuscular pocket where the implant is large and the degree of sagging is greater, or under the breast gland in a subglandular pocket if the implant is small
2. Marking four points on the breast around the areola to determine the amount of skin necessary for both the external skin lining of the new breast and the excess skin in the periareaolar region for the dermal flap to be used for the internal skin lining
3. De-epithelializing the flap to retain the central pedicle
4. Displace the breast subcutaneous down to the level of the pectoral fascia
5. Dissecting the skin on the bias in the upper hemisphere in order to progressively increase the thickness of the subcutaneous fat tissue close to the skin and
6. Dissecting the skin from the parenchymal tissue in the lower hemisphere of the breast
7. Applying a mastopexy prosthesis over the dermal flap in the lower hemisphere to sling the underside of the breast
8. Suturing the mastopexy prosthesis to the pectoralis fascia to promote elevation and shape of the mammary cone
9. Suturing closed the external skin lining while fixing the areolar skin to the external skin lining
10. Dressing the breast in a supportive way that allows drainage of exudates.

Still another preferred embodiment of the invention is a prosthesis for breast reconstruction comprising two or more superimposed, chemically bonded layers of collagenous material which, when implanted into a mammalian patient, undergoes controlled biodegradation occurring with adequate living cell replacement such that the original implanted prosthesis is remodeled by the patient's living cells. The breast reconstruction device of the invention is preferably comprises between three to seven layers of processed tissue matrix, more preferably between four to six layers of processed tissue matrix, wherein it is even more preferred that in these embodiments of the invention that the processed tissue matrix is ICL and wherein the layers are chemically bonded with EDC. The degree of crosslinking is one that allows the processed tissue layers to substantially persist for about six months prior to the layers to bioremodel by infiltration of the patient's cells into the matrix layers to replace the implanted matrix with matrix produced by the patient's cells. Any of the crosslinking methods using EDC, described herein, may be used in the preparation of the breast reconstruction prostheses of the invention. In breast reconstruction surgeries, usually the tissue expander is removed at the six-month timepoint and the implant is put in its place. The breast reconstruction device preferably is meshed by providing an arrangement of slits that communicate between the two planar surfaces of the device.

The mesh is provided as an arrangement of slits running in parallel with adjacent slits offset in staggered arrangement such that when the material is pulled at a direction perpendicular to the direction of the slits, the material will stretch and the slits will open such that the appearance of the material is a mesh. The device material, when pulled in the direction of the slits, still retains its strength as it offers only a minimal amount of stretch. For the surgeon to better conform the device material to the features of the tissue expander or implant, the mesh arrangement allows for certain areas of the material to stretched to open the slits while allowing other areas to remain closed. The mesh arrangement allows for fluid communication between the layers and better conformity to the tissue expander or implant while still allowing it to provide the requisite support and coverage for the expander or implant. Furthermore, the breast reconstruction device of the invention is crescent-shaped, generally wider in the center and narrow on the lateral end portions and is between about 12 cm to about 24 cm long and about 4 cm to about 8 cm wide at its widest point. The end portions may be pointed, rounded or squared-off. The surgeon may select the appropriate sized device from a series of sizes depending on the size of the breast to be reconstructed.

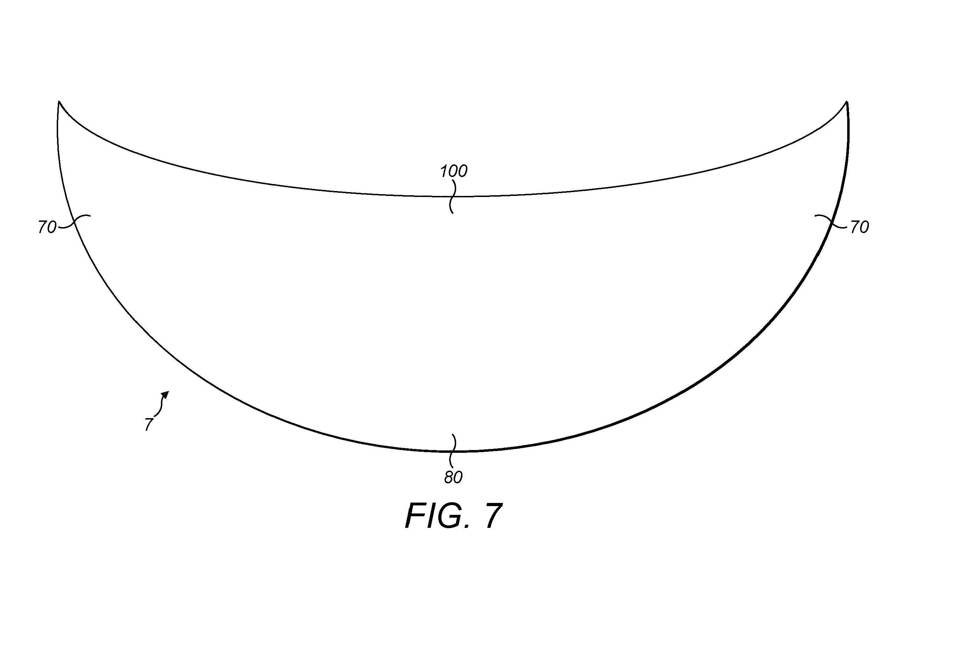

Figure 7 shows a breast reconstruction prosthesis of the invention wherein breast reconstruction prosthesis 7 is elongate and arcuate having two pointed end portions 70 with an apex 80 and base 100.

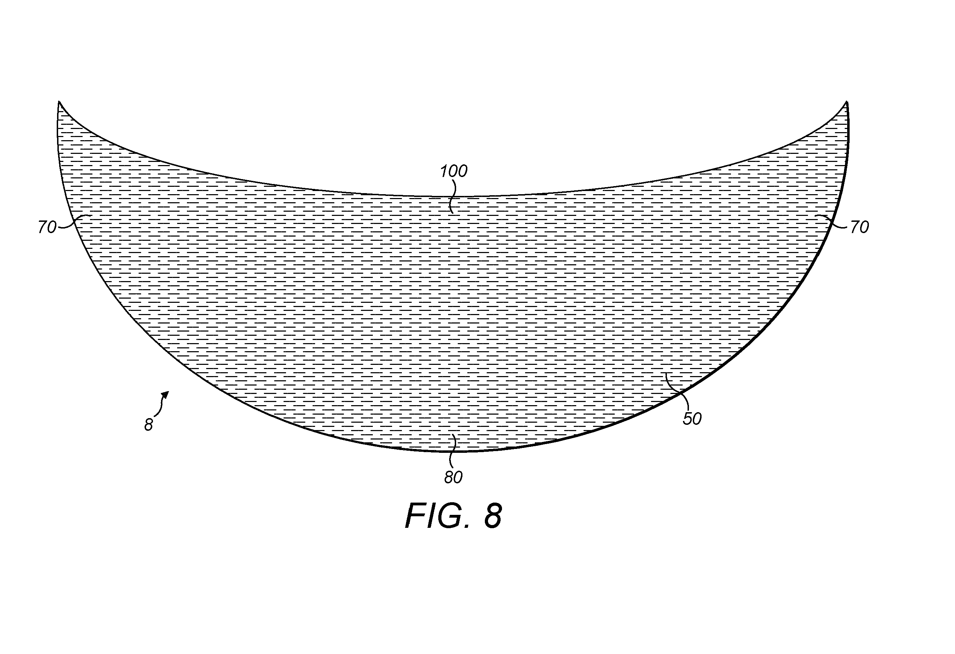

Figure 8 shows a breast reconstruction prosthesis of the invention wherein breast reconstruction prosthesis 7 is elongate and arcuate having two pointed end portions 70 with an apex 80, base 100 and mesh slits 50 in a regular arrangement across the entire prosthesis.

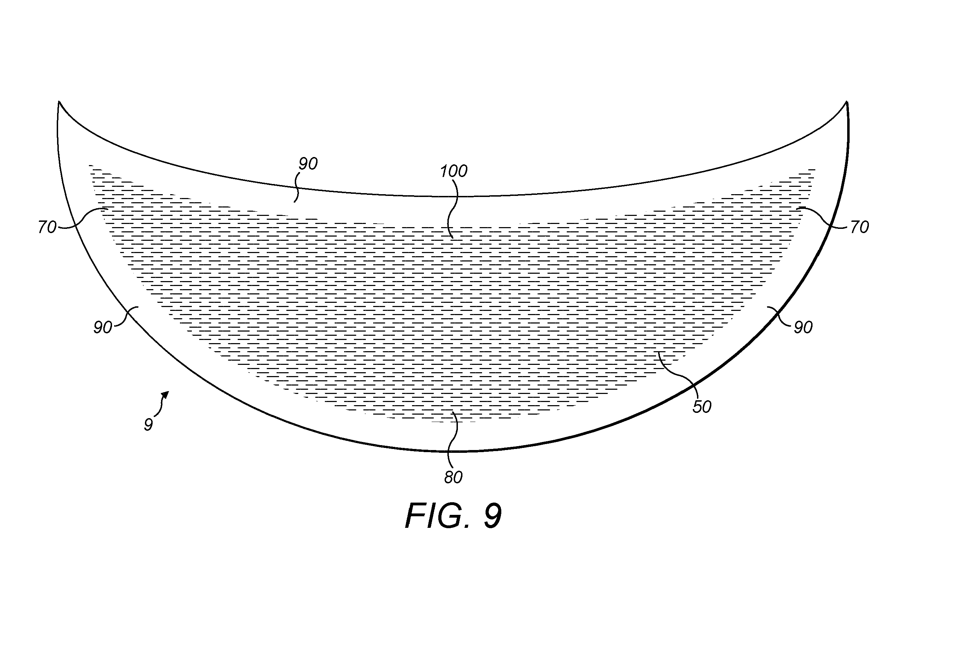

Figure 9 shows a breast reconstruction prosthesis of the invention wherein breast reconstruction prosthesis 7 is elongate and arcuate having two pointed end portions 70 with an apex 80, base 100 and mesh slits 50 in a regular arrangement across the entire device except at the border 90 of the prosthesis.

The following examples are provided to better explain the practice of the present invention and should not be interpreted in any way to limit the scope of the present invention. It will be appreciated that the device design in its composition, shape, and thickness is to be selected depending on the ultimate indication for the construct. Those skilled in the art will recognize that various modifications can be made to the methods described herein while not departing from the spirit and scope of the present invention.

### EXAMPLES

Example 1: Chemical Cleaning of Mechanically Cleaned Porcine Small Intestine The small intestine of a pig was harvested and mechanically stripped, using a Bitterling gut cleaning machine (Nottingham, UK) which forcibly removes the fat, muscle and mucosal layers from the tunica submucosa using a combination of mechanical action and washing using water. The mechanical action can be described as a series of rollers that compress and strip away the successive layers from the tunica submucosa when the intact intestine is run between them. The tunica submucosa of the small intestine is comparatively harder and stiffer than the surrounding tissue, and the rollers squeeze the softer components from the submucosa. The result of the machine cleaning was such that the submucosal layer of the intestine solely remained.

The remainder of the procedure, chemical cleaning according to International PCT Application No. WO 98/49969 to Abraham, et al., was performed under aseptic conditions and at room temperature. The chemical solutions were all used at room temperature. The intestine was then cut lengthwise down the lumen and then cut into 15 cm sections. Material was weighed and placed into containers at a ratio of about 100:1 v/v of solution to intestinal material.
A. To each container containing intestine was added approximately 1 L solution of 0.22 µm (micron) filter sterilized 100 mM ethylenediaminetetraacetic tetrasodium salt (EDTA)/10 mM sodium hydroxide (NaOH) solution. Containers were then placed on a shaker table for about 18 hours at about 200 rpm. After shaking, the EDTA/NaOH solution was removed from each bottle.
B. To each container was then added approximately 1 L solution of 0.22 µm filter sterilized 1 M hydrochloric acid (HCl)/1 M sodium chloride (NaCl) solution. Containers were then placed on a shaker table for between about 6 to 8 hours at about 200 rpm. After shaking, the HCl/NaCl solution was removed from each container.
C. To each container was then added approximately 1 L solution of 0.22 µm filter sterilized 1 M sodium chloride (NaCl)/10 mM phosphate buffered saline (PBS). Containers were then placed on a shaker table for approximately 18 hours at 200 rpm. After shaking, the NaCl/PBS solution was removed from each container.
D. To each container was then added approximately 1 L solution of 0.22 µm filter sterilized 10 mM PBS. Containers were then placed on a shaker table for about two hours at 200 rpm. After shaking, the phosphate buffered saline was then removed from each container. E. Finally, to each container was then added approximately 1 L of 0.22 µm filter sterilized water. Containers were then placed on a shaker table for about one hour at 200 rpm. After shaking, the water was then removed from each container.

Processed ICL samples were cut and fixed for histological analyses. Hemotoxylin and eosin (H&E) and Masson's trichrome staining was performed on both cross-section and long-section samples of both control and treated tissues. Processed ICL tissue samples appeared free of cells and cellular debris while untreated control samples appeared normally and expectedly very cellular.

This single layer material of ICL may be used as a single layer or used to form bonded multilayer constructs, tubular constructs, or constructs with complex tubular and flat geometrical aspects.

### Example 2: Method for Fabricating a Multilayer ICL Construct.

ICL processed according to the method of Example 1 was used to form a multilayer construct having 2 layers of ICL. A sterile sheet of porous polycarbonate (pore size, manufacturer) was laid down in the sterile field of a laminar flow cabinet. ICL was blotted with sterile TEXWIPES (LYM-TECH Scientific, Chicopee, MA) to absorb excess water from the material. ICL material was trimmed of its lymphatic tags from the abluminal side and then into pieces about 6 inches in length (approx. 15.2 cm). A first sheet of trimmed ICL was laid on the polycarbonate sheet, mucosal side down, manually removing any air bubbles, folds, and creases. A second sheet of trimmed ICL was laid on the top facing, or abluminal side, of the first sheet with the abluminal side of the second sheet contacting the abluminal side of the first sheet, again manually removing any air bubbles, folds, and creases. The polycarbonate sheet with the ICL layers was angled up with the ICL layers facing the oncoming airflow of the laminar flow cabinet. The layers were allowed to dry for about 18 ± 2 hours in the cabinet at room temperature, approximately 20 °C. The dried layers of ICL were then peeled off the polycarbonate sheet together without separating or delaminating them and were transferred to a room temperature waterbath for about 15 minutes to hydrate the layers.

Chemical crosslinking solution of 100mM EDC/50% Acetone was prepared immediately before crosslinking as EDC will lose its activity over time. The hydrated layers were then transferred to a shallow pan and the crosslinking agent gently decanted to the pan ensuring that the layers were both covered and free-floating and that no air bubbles were present under or within the constructs. The pan was covered and allowed to sit for about 18 ± 2 hours in fume hood. The crosslinking solution was decanted and disposed. Constructs were rinsed in the pan three times with sterile water for about five minutes for each rinse. Using a scalpel and ruler, constructs were trimmed to the desired size.

Constructs were decontaminated with sterile 0.1% peracetic acid (PA) treatment neutralized with sodium hydroxide 10N NaOH according to US Patent No. 5,460,962,the disclosure of which is incorporated herein. Constructs were decontaminated in 1 L Nalge containers on a shaker platform for about 18 ± 2 hours. Constructs were then rinsed with three volumes of sterile water for 10 minutes each rinse and PA activity was monitored by Minncare strip testing to ensure its removal from the constructs.

Constructs were then packaged in plastic bags using a vacuum sealer which were in turn placed in hermetic bags for gamma irradiation between 25.0 and 35.0 kGy.

### Example 3: Implant Studies Using Multilayer ICL Constructs

New Zealand white rabbits were used for in vivo analysis and all procedures were performed in compliance with Animal Care and Use Committee (ACUC) guidelines. A full thickness defect of approximately two inches was created through the rectus abdominis muscle in each animal and then was repaired with a 6 layer patch prosthesis. Patches were removed at 30, 66, 99 and 180 days post-implant. Three rabbits were sacrificed at each time point and examined for any evidence of herniation, swelling, infection or adhesions. Explanted patches were fixed in formalin and stained with hematoxylin and eosin or alizarin red for histologic evaluation of cell infiltration, inflammatory response and calcification. In some cases, unfixed patches were evaluated to determine the effect of implantation on the mechanical characteristics using uniaxial MTS analysis.

All animals underwent an uneventful post-operative course with no swelling, herniation or inflammation at the repair site of the abdominal wall. At the time of the explant, the inner surface of the patch was covered with a glistening tissue layer that appeared to be continuous with the parietal peritoneum. In one animal explanted after 30 days, a grade one adhesion to the explanted abdominal viscera was seen and appeared to be associated with the suture line rather than the implant itself. Neovascularization of the peritoneal surface of the patches was observed at all time points.

Within 30 days, the peritoneal surface of the patch was covered with mesothelium. Inflammatory cells typical of a foreign body response were present throughout the explant but more prevalent at the periphery of the patch. The inflammatory cells consisted mostly of macrophages and multinucleated giant cells with fewer lymphocytes, heterophils and fibroblasts. After implantation for 66 days, the histology was similar but with fewer inflammatory cells. In addition, the patches had begun to incorporate into the native abdominal wall tissue. At 99 and 180 days, infiltration of host fibroblasts was apparent by hematoxylin and eosin staining and by Masson trichrome staining. Alizarin red staining for calcium showed that there was no evidence of calcification in the patch material. Small focal areas of calcification were associated with the suture material.

Mechanical Testing was performed at the time of explant to determine the ultimate tensile strength (UTS) of the construct. Briefly, the tissue was excised leaving approximately 1 inch of surrounding tissue from the edges of the construct. The surrounding tissue at opposite ends of the construct was then gripped and pulled to failure in uniaxial tension at a constant strain rate of 0.013 s⁻¹ using a servohydraullic MTS testing system with TestStar-SX software. The UTS was then calculated from the peak force. All failures occurred within the tissue region of the testing specimens, suggesting that the construct was equal to or stronger than surrounding tissue, was well integrated into surrounding tissue, and maintained sufficient strength in its performance as a hernia repair patch.

The combination of mechanical properties and potential for good integration into the host tissue make the ICL a promising material for soft tissue repair. These studies have shown that the formation of adhesions is minimal and there is no indication of calcification in the patches. Preliminary analysis of the mechanical characteristics suggests that this collagen construct can maintain the necessary strength while remodeling and incorporating into the surrounding tissue. This ability of the patch to remodel provides an advantage over prosthetic materials that do not integrate well into the surrounding tissue.

### Example 4: Mechanical testing techniques and properties of Multilayer ICL Prostheses

Preferred embodiments of multilayer ICL patch constructs formed by the method of Example 3, including gamma irradiation were tested. Constructs of 2, 4, and 6 layers of ICL crosslinked with 100 mM EDC in 50% Acetone (100/50) and 6 layer constructs with crosslinked with 7 mM EDC/90% acetone v/v in water (7/90) and 1 mM EDC in water (1/0) were evaluated along a number of measures. Results are summarized in Table 1.

Tensile failure testing was performed using a servohydraullic MTS testing system with TestStar-SX software. Strips 1.25 cm in width were pulled to failure in uniaxial tension at a constant strain rate of 0.013 s⁻¹. The slope of the linear region (Ey) and the ultimate tensile strength (UTS) were calculated from the stress strain curves.

The adhesion strength between the layers was tested using a standard protocol for the testing of adhesives (ASTM D1876-95). The adhesion strength is the average force required to peel apart two layers of laminated ICL at a constant velocity of 0.5 cm/sec.

A differential scanning calorimeter was used to measure the heat flow to and from a sample under thermally controlled conditions. The shrink temperature was defined as the onset temperature of the denaturation peak in the temperature-energy plot. Suture retention was not performed on 2 or 4 layer constructs cross-linked in 100 mM EDC and 50% acetone since the suture retention (3.7N ± 0.5 N) for a 2 layer construct cross-linked in 1 mM EDC and no acetone (much less cross-linked) was well above the 2 N minimum specification. Lamination strength between ICL layers and shrinkage temperature are dependent on the crosslinking concentration and the addition of acetone rather than the number of layers in a construct.

| Table 1: Mechanical Properties of Multilayer ICL Constructs | | | | | |
|---|---|---|---|---|---|
| Mechanical Analysis | 2 Layer 100 mM EDC/ 50% Acetone | 4 Layer 100 mM EDC/ 50% Acetone | 6 Layer 100 mM EDC/ 50% Acetone | 6 Layer 70 mM EDC/90% Acetone | 6 Layer 1 mM EDC in Water (no acetone) |
| Ultimate Tensile Strength (N/mm) | 0.6 ± 0.1 | 3.1 ± 0.2 | 2.0 ± 0.2 | 2.7 ± 0.2 | 1.3 ± 0.4 |
| Young's Modulus (MPa) | 38.0 ± 5.8 | 49.5 ± 4.0 | 35.9 ± 2.6 | 43.0 ± 1.2 | 14.5 ± 7.8 |
| Lamination Strength (N/m) | 39.7 ± 6.1 | | | 63.1 ± 24.4 | 8.1 ± 2.1 |
| Suture Retention (N) | not tested | not tested | 6.6 ± 1.6 | 10.6 ± 2.2 | 10.9 ±2.8 |
| Shrink Temperature (°C) | 72.5 ± 1.1 | | | 69.5 ± 0.1 | 64.0 ± 0.2 |

### Example 5: Method for Treating an Individual With Intrinsic Sphincter Deficiency Using an ICL Construct as a Sling

Patients, mostly women patients, who have intrinsic sphincter deficiency (urinary incontinence) with coexisting hypermobility of the bladder and are treated with a sling have a high rate of cure or improvement depending on the extent of complications. The sling procedure stabilizes the anatomic support and compresses the urethra.

A bonded multilayer ICL construct between 2 and 10 layers is formed according to the method of Example 2 is used as a sling in these procedures.

Under a plane of anesthesia, the operation is performed through an abdominal approach, a vaginal approach, or a combination of both, depending on the chosen implant procedure. Procedures differ in how the sling is placed under the urethrovesical junction and is anchored. Anchoring points include retropubic or abdominal structures, or to both.

Retropubic suspension procedures include several different techniques performed through a low abdominal incision, particularly for the retropubic anchoring approach. However, all techniques have in common elevation of the lower urinary tract, particularly the urethrovesical junction within the retropubic space. The techniques do differ, however, in what structures are used to achieve the elevation.

In the Marshall-Marchetti-Kranz procedure, the periurethral tissue is approximated to the symphysis pubis. In the Burch colposuspension method, the vaginal wall lateral to the urethra and bladder neck is elevated toward Cooper's ligament. The paravaginal repair involves reapproximating the endopelvic fascia to the pelvic wall at the arcus tendineus.

### Example 6: Method for Treating an Individual With Rectocele

Rectocele is herniation of the rectum into the vagina causing disruption of bowel function and pain. The rectocele is usually occurs in aging women through weakening of the wall between the rectum and the vagina.

A bonded multilayer ICL construct between 2 and 10 layers is formed according to the method of Example 2 and is surgically implanted an sutured in the rectovaginal space to provide support to the rectocele by suspending the rectum in its natural position. As the construct works with the body's natural tissue to support the rectum, it bioremodels and becomes a part of the existing tissue to thus recreate a natural support tissue.

### Example 7: Method for Treating an Individual With Vault Prolapse

Vault prolapse is when the vaginal apex descends from its natural anatomical position. The condition sometimes occurs in women following hysterectomy or with aging.

The procedure to remedy the condition is called sacrocolpopexy. In the procedure, a bonded multilayer ICL construct between 2 and 10 layers is formed according to the method of Example 2 and is attached to the sacrum and the vaginal cuff thus providing support for the vaginal vault. The ICL construct stabilizes the apex to hold it in the correct anatomical position. The construct, while supporting the tissue, performs a dual role. First, it creates a support to prevent recurrence of prolapse and second, it bioremodels to integrate with the body's natural tissue.

### Example 8: Method for Treating an Individual With Cystocele

A cystocele is a type of tissue herniation that occurs between the urinary bladder and the vagina where the tissue wall allows the bladder to fall into the vagina to some extent. The cystocele condition occurs with a weakening of the separating tissue, usually with age. With this condition, some patients experience a painful condition called dyspareunia.

The procedure for repairing the cystocele involves implanting a bonded multilayer ICL construct between 2 and 10 layers formed according to the method of Example 2 is used to support and stabilize the urinary bladder. The construct is placed along the tissue wall between the bladder and the vagina with securely attached using sutures at the arcus tendinus. Once in place, the ICL construct provides reinforcement to the tissue between the vagina and the bladder while it bioremodels to integrate with the body's natural tissue.

### Example 9: Method for Repairing Dura Mater

The dura mater is the tough fibrous membrane that encases the brain and the spinal column. As an outer covering of the meninges, this is the fibrous sheath that encircles the central nervous system. It performs two functions, first, to keep the spinal fluid in, and, second, to stop infection from getting into the central nervous system. Surgical procedures or trauma that breach the dura mater may result in a hole, that because of the fibrous, inelastic nature of dura, may not be possible shut by primary closure. To seal the nervous system in such a situation, a multilayer ICL construct is used to restore and replace the dura mater.

Animals are anesthetized, entubated and appropriately positioned to access the cranium. The scalp is shaved, and local anesthesia (1% lidocaine) is administered. Through a midline scalp incision and following incision of the fascia at the superior temporal line, the temporalis muscle is elevated laterally to expose the parietal convexity. A temproparietal craniotomy is made with and electric drill and burr. Bleeding bone edges are waxed. The dura mater is resected at the craniotomy sites under loop magnification while care is taken to avoid injury to the underlying cerebral cortex.

A bonded multilayer ICL construct between 2 and 10 layers is formed according to the method of Example 2 is trimmed and placed above the cerebral cortex and sutured with a nylon suture. The craniotomy flap is replaced and the wound is irrigated with saline and stapled closed. Antibiotic ointment and a sterile dressing are applied and the dog's heads are protected using an Elizabethan collar. The animals are monitored and administered with antibiotic, anesthesia and dressing changes.

At various staggered timepoints after the surgery, dogs are sacrificed, tissue is cut to include all tissues between the scalp and the cerebral cortex are fixed, sectioned, and stained on glass slides.

While there is some minor inflammation observed, it is likely due to the trauma of surgery. Neovascularization is observed but no evidence of graft rejection or humoral response is noted. In later timepoints, less inflammation and some bioremodeling is observed.

### Example 10: Method for Treating a Wound

Either a single sheet layer of ICL from Example 1 or a bonded multilayer sheet construct of ICL formed by the method of Example 2 is used to treat a full-thickness skin wound. The sheet is meshed or fenestrated to create small openings to allow for seepage of wound exudate.

Skin wounds including second degree burns, lacerations, tears and abrasions; surgical excision wounds from removal of cancerous growths or autograft skin donor sites; and skin ulcers such as venous, diabetic, pressure (bed sores), and other chronic ulcers are managed using ICL in single or multilayer form. The collagenous ICL matrix protects the wound bed while maintaining moisture and allowing drainage from the wound. Before the ICL is applied to the wound, the wound bed is prepared for its application.

Patients with burn wounds requiring grafting are selected. ICL is placed either directly on the excised wound bed or over meshed autograft unexpanded or expanded at a ratio of 2:1 or more. Test sites (ICL) and control sites (autograft), when used, are of the same mesh ratio. The burned wound sites to be grafted are prepared, such as by debridement, prior treatment according to standard practice so that the burned skin area was completely excised. Excised beds appear clean and clinically uninfected.

Patients undergoing surgical excision are locally anesthetized. The pre-operative area is cleansed with an anti-microbial/antiseptic skin cleanser (Hibiclens®) and rinsed with normal saline. Deep partial thickness wounds are made in the skin and the skin is grafted elsewhere unless it is cancerous. ICL is applied to the wound bed and sterile bandages are applied.

In either wound case, appropriate post-operative care is provided to the patient in examination, cleaning, changing bandages, etc. of the treated wounds. A complete record of the condition of the treated sites is maintained to document all procedures, necessary medications, frequency of dressing changes and any observations made. The wound beds remain protected from the external environment and moist to aid in wound management and healing.

The wound dressing was tested in an animal model. The wound dressing construct of the invention is either a single or multilayer sheet construct made from ICL formed by the methods of example 1 and, if a multilayer construct, by the methods of examples 1 and 2. A rat full-thickness wound healing model (a commonly used model for wound dressing products) was used to assess the performance of a wound dressing construct made from a single layer material of ICL. A total of 20 animals, four per evaluation timepoint, had two (2) 2 cm x 2 cm full-thickness excision wounds created on their dorsum. The test and control articles were cut slightly larger than the wound periphery and applied dry to either wound following a randomized application scheme. The dressings were rehydrated by the wound fluid and sterile saline as necessary. Secondary dressings of petrolatum gauze were applied over each test and control article and changed weekly or at each evaluation timepoint. The wounds were assessed at 3, 7, 14, 28 and 42 days post-treatment. Assessments included rate and percentage wound closure (based on wound tracings), erythema, exudate and histology of explanted wound sites.

According to the results of the analysis of the percentage and rate of wound closure, the wound dressing construct treated sites demonstrated slightly faster, although not statistically significant, wound closure than the control sites. The analysis of time to complete wound closure did not find a difference between the test and control treated sites. The results of the erythema, exudate and histology analyses were equivalent for the two products. Histology showed that the wound dressing construct made from single layer ICL exhibited requisite healing characteristics over time, re-epithelialization of the wound, and resorption of the collagen materials. There was no evidence of an adverse reaction to the construct by the test subjects.

### Example 11: Method for Repairing a Hernia

A hernia is a tear or hole in the musculature of the abdominal wall through which the intestines bulge out, producing a lump in the skin tissue. Inguinal hernias occur through a hole in a flat tissue surface; femoral hernias are an uncommon type of groin hernia in which a patient's intestine pushes through the abdomen via a femoral tunnel. Surgery is s performed under local anesthesia and may be performed laparoscopically.

To repair an inguinal hernia, a bonded multilayer ICL construct between 2 and 10 layers is formed according to the method of Example 2 is used to patch over the hole opening. The construct is sutured along the edges of the entire area of the groin that is susceptible to hernia formation to prevent further herniation or recurrence.

The repair of a femoral hernia involves plugging a tunnel, the ICL construct can be folded to form a plug, (similar to the corking of a bottle). The ICL plug closes off the tunnel and is sutured in place.

The ICL is bioremodelable and is infiltrated with patient's cells that replace the ICL matrix with new endogenous matrix from the cells while performing the physical function of buttressing and reinforcing the tissue wall.

### Example 12: Method for Rotator Cuff Repair

Rotator cuff tears are broadly classified as crescent-shaped (or U-shaped for extensive crescent shaped) tears or L-shaped tears and such tears occur at the tendon-bone junction at the top of the humerus bone. Usually, the tendon is sutured back to the bone directly or sometimes with the aid of a suture anchor (as in crescent-shaped tears). A multilayer bioengineered flat sheet ICL prosthesis is used to augment the suture line in such repairs and to reinforce or replace extensively damaged tendon tissue in the repair of the bone-muscle complex. After the tendon is sutured to the bone, the ICL is overlaid and sutured to the tendon to reinforce the tendon to prevent recurring tears or suture pull-out.

### Example 13: Use of a Bioengineered Flat Sheet ICL Prosthesis to Repair the Annulus Fibrosis After Partial Discectomy

A bioengineered flat sheet ICL prosthesis prepared according to the method of either Examples 1 and 2 are implanted in pigs to demonstrate the use of the material to repair the annulus fibrosis after partial discectomy.

Six young pigs of either sex up to 50 kg are housed individually for a minimum of two days prior surgery while fed with standard pig chow.

Experimental animals are pre-anesthetized with Telazol and atropine and intubated. The are placed on inhalation gas of isoflurane and oxygen and kept in surgical plane of anesthesia. They are also administered an antibiotic.

Defects in the discs are created by making a 5 x 10 mm incision in the annulus followed by a standard discotomy with equal nuclear removal at each space. A total of three discs are operated on per pig. Two sites are treated with the bioengineered flat sheet ICL prosthesis and the remaining site serves as a control. To apply the bioengineered flat sheet ICL prosthesis, it is first trimmed into three or four smaller pieces and then inserted into the annular hole opening. Two animals are euthanized on each of weeks 2, 4, and 6 and the surgical sites are removed. The discs are placed in formalin and then 70% ethanol prior to histological processing.

### Example 14: Use of a Bioengineered Flat Sheet ICL Prosthesis With an Intervertebral Disc Spacer to Maintain the Intervertebral Space.

To demonstrate the use of the bioengineered flat sheet ICL prosthesis with an intervertebral disc spacer, experiments are conducted in a pig model. A single layer ICL sheet formed according to the method of Example 1 or a bonded multilayer ICL construct between 2 and 10 layers formed according to the method of Example 2 is used in this study.

Six young pigs of either sex up to 50 kg are housed individually for a minimum of two days prior surgery while fed with standard pig chow.

Experimental animals are pre-anesthetized with Telazol and atropine and intubated. The are placed on inhalation gas of isoflurane and oxygen and kept in surgical plane of anesthesia. They are also administered an antibiotic.

Defects in the discs are created by making a 5 x 10 mm incision in the annulus fibrosis followed by a standard discotomy with equal nuclear removal at each space. A total of three discs are operated on per pig.

Through the hole made in the annulus fibrosis, the intervertebral space is opened and the disc is removed, restricted to the anterior and middle third portion. The intervertebral disc spacer comprising Dacron mesh and hydrogel is placed into the thoracic cavity by passing it through the hole in the annulus fibrosis. The good position of the implant is ascertained using radiologic procedures and then the spacer is then fixed into place. The bioengineered flat sheet ICL prosthesis is then applied to the annular opening by first trimming the construct to the size of the annular hole opening and then sutured to the tissue surrounding the opening of the space using resorbable sutures. While all three sites are provided with an intervertebral disc spacer, two sites are treated with bioengineered flat sheet ICL prosthesis and the remaining site serves as a control.

Two animals are euthanized on each of weeks 2, 4, and 6 and the surgical sites are removed. The discs are placed in formalin and then 70% ethanol prior to histological processing. The discs are serially sectioned and examined under microscope to gauge healing and bioremodeling.

In all specimens, the intervertebral disc spacer maintained its original placement. In control specimens, the nucleus pulposus shows a significant loss of proteoglycans and collagen and an increase in other non-collagenous proteins in the cavity. In experimental specimens, the connective tissue construct forms a complete scar over the opening made in the annular fibrosis. The biochemical make up of the cavity had changed somewhat but was closer to composition of the negative control specimens, indicating that fibrosis of the cavity had been substantially prevented by the closure of the annulus after disc injury.

### Example 15: A Time Course Study Using Bioengineered Flat Sheet ICL Prosthesis in Annulus Fibrosus Repair of Pigs

Discectomy to remove ruptured and expulsed nucleus pulposus is a common clinical practice to relieve pain and neurologic disturbance. The procedure creates a defect in annulus fibrosus that is often filled by fibrotic tissues, a situation that eventually leads to collapse of the intervertebral disc and requires fusion of the adjacent vertebral segments.

Single and multilayer bioengineered flat sheet ICL prostheses are prepared according to the methods of Examples 1 and 2. The purpose of this study was to evaluate the feasibility of bioengineered flat sheet ICL prosthesis for repair of the annulus fibrosus in a porcine model and to determine the biocompatibility, persistence and remodeling of the constructs in this model.

Six 3 to 4 month-old pigs were used for the study. Three consecutive vertebral discs are posteriorly exposed through a laminotomy approach for each animal. A surgical annular defect are created in each exposed disc. Several pieces of connective tissue construct, each about 2 to 5 mm in diameter, are implanted into two defects of each animal. The other disc defect is left empty as a control. The pigs are euthanized, in groups of two, at two, four and six weeks post implant. The vertebral columns containing operated discs are removed and fixed in 10% neutral buffered formalin. Bright field microscopy is done on hemotoxilin and eosin stained sections for general evaluation.

Microscopy reveals clear evidence of implanted bioengineered flat sheet ICL prosthesis remnants in several of the treated annuli from the two animal groups euthanized at two and four weeks. There was also identifiable remodeling of the connective tissue construct remnants by the host tissue. The implanted defects show less inflammation and more advanced healing than controls at all time points. The implant areas have cartilaginous tissue bridging the opening, whereas the control defects still have a significant amount of fibrotic tissue. The results from this feasibility study indicate that the implanted pig connective tissue constructs are biocompatible to the host tissue and enhance reparative activities of the annulus.

### Example 16: Rabbit Soft Tissue Defect Repair Studies

A study was conducted to determine the in vivo performance of multilayer ICL constructs as a surgical mesh/patch product. New Zealand white rabbits were used for the *in vivo* implant studies. A full thickness defect of approximately 5 cm long was made in the anterior abdominal wall through the center of the rectus abdominis muscle and the underlying peritoneum. This is a widely accepted model for the evaluation of surgical mesh/patch products.

A six layer ICL construct crosslinked with 100 mM EDC was tested for implant periods ranging from one to six months with three rabbits evaluated at each time point (30, 60, 99, 180 days). Minimal adhesion formation was observed at the selected timepoints. The chemically cleaned surgical mesh became well integrated with the host tissue along the suture line as shown by histology and the lack of suture line herniation. There was a moderate inflammatory response that subsided after several months, and no evidence of calcification of the implants was detectable. At six months there was little degradation of the implants. Mechanical testing of the explanted patch constructs demonstrated that there was no significant difference in the strength of the patch/abdomen complex at 180 days post-implant (27.8 ± 5.6 N/cm) and the control host abdominal wall (28.1 ± 14.6 N/cm), indicating that the prosthetic material retains its strength characteristics when used to treat a host. These results confirmed the suitability of the chemically cleaned, multilayer ICL constructs for surgical repair applications.

The low immunogenicity of the chemically cleaned porcine Type I collagen was demonstrated in this study by analyzing the antibody response of rabbits that received the porcine intestinal collagen surgical mesh. ELISA analysis of serum samples taken from grafted rabbits showed little or no production of antibodies to Type I porcine collagen relative to normal rabbit serum. This lack of response was confirmed by Western blot analysis using purified porcine Type I collagen.

A second *in vivo* study using the rabbit soft tissue defect model evaluated the performance of four layer ICL constructs crosslinked with 1 mM EDC. These patches were implanted in the rabbit model for three months. The gross examination at explant showed results similar to the previous studies. The patches were integrated with the host tissue with no evidence of either seroma or adhesion formation. However, the lower crosslinking did allow faster remodeling than higher crosslinked constructs. There was a substantial amount of cellular infiltration and remodeling of the collagen of the ICL construct after 90 days. There was no herniation or other functional failure of the grafts throughout the course of the study. Thus, even under conditions in which ICL constructs are remodeled and replaced by host tissue, their repair functions do not appear to be compromised.

### Example 17: Calcification Study

A feature of the ICL constructs is that they do not elicit calcification of the ICL material as is common with some types of collagenous implants. EDC crosslinked ICL was compared to glutaraldehyde crosslinked heart valves, which will undergo calcification when implanted.

A one layer porcine intestinal collagen sheet crosslinked with 1 mM EDC and gamma irradiated (25-35 kGy) was evaluated in a juvenile rat calcification model. The collagen material was implanted subcutaneously between the skin and the rectus abdominis muscles. Bovine heart valves fixed with glutaraldehyde were implanted subcutaneously between the skin and the rectus abdominis muscles as the positive control. Calcification was assessed by Alizarin Red Staining. All six of the rats that received glutaraldehyde treated valve leaflets showed extensive calcification as determined by Alizarin Red staining. In contrast, even after 28 days, no calcification was detectable in the porcine intestinal collagen.

### Example 18: Comparative Study of ICL Prostheses with Other Tissue-Derived Products

This study is designed, in a canine model, to evaluate the performance of various tissue-derived materials for soft tissue defect repair under loads similar to those that would be experienced in clinical situations. Cadaveric dermis and similar decellularized human dermis derived products (e.g., LifeCell AlloDerm), as well as fascia lata derived products are used clinically for numerous soft tissue repair applications such as pubovaginal slings and reconstructive procedures. Cadaver grafts (human fascia lata), xenogeneic tissue (bovine pericardium) and synthetic fabrics have been evaluated as soft tissue substitutes. Use of cadaver tissues is limited by fear and transmission of infectious disease while the use of synthetic material is associated with implant encapsulation, adhesion formation and foreign body reactions. These materials have been used world wide until there was a concern for the transmission of fatal diseases such as Creutzfeldt-Jakob disease (CJD), Autoimmune deficiency syndrome (AIDS) and bovine spongiform encephalopathy (Mad Cow disease). Other issues such as calcification, adhesions, antigenicity and inability to integrate with surrounding tissue (which can lead to re-herniation of the repaired defect have led to the search for more natural collagenous materials). The purpose of this study is to evaluate the use of ICL constructs material for soft tissue reinforcement in comparison with materials currently used in clinical applications.

Surgical soft tissue repair materials were tested in an animal (canine) full-thickness rectus abdominis replacement model to integrate into the host tissue and the feasibility of the test materials to become a scaffold for remodeling into functional rectus abdominis. This study is being designed to provide in vivo comparison data regarding the safety and efficacy of a surgical patch material derived from a Type I collagen biomaterial fabricated from the submucosa of porcine small intestine. The specific aims are to evaluate the difference between two different ICL surgical patches (high and low crosslinking) and that of commercially available soft tissue reinforcement and sling products such as cadaveric dermis (Boston Scientific) and cadaveric fascia lata (Mentor). The canine rectus abdominis model has been selected for this study because the abdominal anatomy and biomechanical stresses are similar to that of humans, and it is an accepted and widely used model for hernia and soft-tissue repair.

Two ICL construct designs (5 layer laminates, with either a low or high level of collagen crosslinking) were tested: Highly crosslinked constructs were crosslinked in 10 mM EDC/90% acetone in water and low crosslinked constructs were crosslinked in 1 mM EDC in water. The order of implant operation and study group designation (animals euthanized at 1, 3, 6 or 12 months) were randomized. Additionally, for each animal the implant location of the test and control materials were randomized. A valid and unbiased randomization methodology was employed.

Each animal was pre-medicated with butorphenol (0.2 mg/kg), acepromazine (0.1 mg/kg), and atropine (0.02 mg/kg) IM, an intravenous catheter was placed in the cephalic vein, and propofol was administrated 10-15 minutes later at a dose of 4 mg/kg IV at a rate of 1 ml/10 kg/min or to effect. The animal was immediately intubated and initially maintained under anesthesia with inhalant isoflurane anesthetic at 2.5 - 4%, and 0.5 - 2.5 % for maintenance delivered through either a volume-regulated respirator or rebreathing apparatus. If emergency drugs were needed they were administered through IV line and the drug, dose, route, and site of administration will be documented in the surgical file. IV fluids (Lactated Ringers) were administered throughout the surgical procedure at 10 mls/kg/hr. Cefazolin, an anitbiotic, was given prior to surgery (17 mg/kg IM).

Once anesthetized, the abdomens of the dogs were shaved. The operative area was cleaned with a three alternating scrubs of povidone-iodine scrub and 70% alcohol, once the alternating scrubs were done a final application of povidone-iodine solution was applied and allowed to dry and the area was draped for aseptic surgery. The animal was placed in the supine position and aseptically prepped and draped. A skin incision approximately 20 cm long was performed, which was 2.5 cm lateral to the linea alba and carried down to the anterior abdominal wall. The skin was carefully undermined to separate it from the abdominal wall fascia, exposing an area approximately 4 cm by 20 cm, adequate exposure for 2 implant sites. A full thickness defect measuring approximately 3 cm by 5 cm was made in the anterior abdominal wall (2.5 cm above the level of the umbilicus) through the rectus abdominis fascia, muscle and underlying peritoneum. The implant material (test or control) was trimmed to the size of the defect and attached to the edge of the defect with continuous uninterrupted non-resorbable 3-0 Prolene suture.

In a similar manner, three additional defects (for a total of four defects) were created and implant material (test or control) was sutured into place. The second defect was created through the initial skin incision (positioned 2.5 cm below the level of the umbilicus), whereas two additional defects were created in a similar manner through a second contralateral skin incision, 2.5 cm from the linea alba. The underlying tissue was sutured with 2-0 Vicryl and the skin was closed with a 3-0 Vicryl sutures. Each defect was equally spaced 5 cm apart, centered around the umbilicus (two defects 2.5 cm to the left of the linea alba, two defects 2.5 cm to the right of the linea alba.)

Animals were allowed to recover from anesthesia, and extubated when the swallowing reflex had returned. In addition to the preoperative administration of Butorphenol (0.2 mg/kg, IM), a 3 day post surgery regimen of Buprenorphine @ 0.02 mg/kg Bid SC was given. Cefozolin was given at a dose of (17 mg/kg) IM BID for 3 days post surgery. On Monday Wednesday and Friday of the first two weeks following surgery, and once a week thereafter, all sites were palpated to determine if reherniation has occurred and all sites were viewed to determine if graft rejection or infection have occurred.

X-rays of implant area were taken (before explant) at the 6-month timepoint to evaluate calcification of implants. Each patch was removed, en bloc, with at least 2 cm of adjacent host tissue. The explant was sectioned into two equally sized segments in the anterior/posterior direction. One segment was placed in cold saline (for mechanical testing) and the second in 10 % formalin (for histological processing). A body wall segment was removed for a control for the mechanical testing. This segment was 5 cm in width and removed from the tissue between the patch and the midline. Two sections were removed and saved in cold saline for testing.

### Gross observations at necropsy:

At one month, all of the sites that were observed in each animal showed no evidence of re-herniation, infection or hemorrhage. All material implanted was present at explant and easily identifiable from host tissue.

At three months, all of the sites that were observed in each animal showed no evidence of re-herniation, infection or hemorrhage. All material implanted was present at explant and easily identifiable from host tissue.

At six months, all of the sites that were observed in each animal showed no evidence of re-herniation, infection or hemorrhage. The highly crosslinked ICL patches were easily identifiable and still completely intact. The low crosslinked ICL patches were identifiable, but were well adhered and incorporated into the surrounding host tissue. The cadaveric fascia lata was not easily identifiable and the remaining tissue (when found) appeared stringy and necrotic. The cadaveric dermis (when present) appeared spongy and necrotic. The cadaveric dermis had changed color, to a dark yellowish brown.

### Histology review:

The test patches made from ICL were comprised of two basic morphologies: a linear dense eosinophilic material apparently comprised of a collagen material, and a wide linear band of collagenous material differing from host collagen by its relative acellularity and tinctorial staining. ICL articles were easily identified in all patch samples. The cadaveric dermis samples were present at one month and in only one of three samples at 6 months. Some host cellular infiltration of the cadaveric test articles was seen at the one-month sacrifice. By the three-month sacrifice, as mentioned above, remodeling consisted of increased fibroblast infiltration of most of the cadaveric patches with comparable or less mixed inflammatory cell infiltration than seen at the one month sacrifice. Mixed cell inflammatory infiltration consisted of polymorphonucleated cells and macrophages along with other cell types. By six months only one cadaveric dermis patch was identified and it was mildly infiltrated around its perimeter by fibroblasts and mononuclear inflammatory cells.

No cellular infiltration of the ICL test articles was seen at either one or three months, but there was an increased amount of lamellar splitting and cellular infiltration of the lower crosslinked ICL test articles at three and six months compared to one month. Cellular infiltration consisting mostly of fibroblasts was observed in the lower crosslinked ICL patches at six months. All patch sites, whether test article was specifically identified or not, were surrounded by a prominent host tissue response of fibroplasia and/or fibrosis. By three months, fibrosis was always present and fibroplasia was uniformly severe.

At 6 months, fibrosis was generally severe and fibroplasia generally ranged slight/mild to severe. The decrease in severity of fibroplasia at 6 months with overall increased severity of fibrosis as compared to the 3 months was interpreted as a shift to a more mature host tissue reaction. This was interpreted as a normal healing response, i.e., scarring, of the higher crosslinked ICL patches. In all cases, test article patches performed the expected function of closure of an abdominal defect. All test articles appeared to be compatible with the host tissue. There was variation in the remodeling of the test articles by host derived cells with more degradation than remodeling appearing to be more advanced in the cadaveric patches. Degradation of the cadaveric tissues was seen as areas of granularity in the wide collagenous band material and occurred in only one of the cadaveric patches at one month and minimally in two of six (one ion each type of graft) at three months. Degradation was not seen in the one identifiable cadaveric dermis patch at six months. None of the patch types appeared to be undergoing substantial degradation other than that which accompanies remodeling; in other words, there was no evidence of excessive phagocytosis of test article materials by macrophages and/or giant cells and no calcification of the patches observed. By six months the lack of identifiable cadaveric fascia lata and 2 of 3 cadaveric dermis patches was interpreted as advanced remodeling of the patches by host tissue. The foreign body granulomatous inflammation seen in many of the patches at one, three, and six months was considered a reflection of the surgical procedure rather than a reaction to an inherent quality of the test articles.

### Example 19: Circumareolar Mastopexy using Bioengineered Collagen

An inferior suspensory hammock was used to elevate the parenchyma and prevent inferior fallout. In order to diminish seroma formation and improve subcutaneous adherence, the mastopexy prosthesis should be perforated or meshed. This can be accomplished either by hand or with the use of a skin graft mesher.

A previously marked region within the incision was depithelialized. Care was taken to remove equal amounts of pigmented and unpigmented skin. A subcutaneous dissection to the pectoralis muscle is performed from the two o'clock to ten o'clock position. The mastopexy prosthesis was then secured to the pectoralis fascia using permanent suture. A series of interrupted sutures were used to tack the edges of the hammock to the breast tissue along the entire lower pole of the breast. Roughly 1 to 2 cm of breast parenchyma projected from the most anterior portion of the hammock. This allowed for an unencumbered, periareolar closure. To close the periareolar scar, 3-0 Goretex suture on a Keith needle was utilized in subdermal plane and was secured around a 42 mm disc. The periareolar skin was then closed in the standard fashion using 4-0 vicryl suture in the dermis and 5-0 chromic gut, half-buried, mattress suture in the skin.

This treatment has shown good maintenance of contour and projection, and no incidence of infection or extrusion.

### Example 20: Complete Inferior Expander Coverage With A Breast Reconstruction Prosthesis Using Bioengineered Collagen

The breast reconstruction prosthesis, referred to herein as "BCP", of the invention is used to provide complete coverage of an expander or an implant in post-mastectomy breast reconstruction. The BCP comprises four layers of ICL that are chemically bonded by crosslinking with EDC to a degree that the prosthesis is intended to persist up to six months prior to bioremodeling, wherein the BCP is a crescent shape and provided with an arrangement of slits that form a mesh. The following method for covering an expander with a BCP may also be applied to an implant. The pectoralis major muscle deficit encountered inferiorly in expander reconstructions is supplemented by BCP, thereby providing complete coverage and support of the at the breast lower pole. To determine the appropriate size of BCP to be used intraoperatively, the length of the arc between the inframammary fold ("IMF") and the lateral mammary fold ("LMF") is measured and marked, prior to mastectomy, to determine the size of the BCP needed for post-mastectomy reconstruction. Post-mastectomy, the pectoralis major muscle is elevated along its inferior border. The IMF and the LMF may be re-attached and stabilized at this stage as the BCP will be placed at the arc site and the attachment point of the BCP will recreate these landmarks. The recreation of the LMF with the BCP will spare the serratus anterior muscle that is traditionally used in recreating the LMF. The BCP is placed at the arc site facing the mastectomy skin flap and is held temporarily in place with sutures at each end to hold the BCP in place while it is being centered over the arc site. After centering the BCP over the arc, at the desired IMF, the BCP is anchored inferiorly at several points. The tissue expander is inserted under the pectoralis major muscle and the BCP is then maneuvered over the expander and temporarily stapled to the pectoralis major muscle. After repositioning, the BCP is sutured to the pectoralis major muscle under appropriate or minimal tension while removing the temporary staples. The expander is deflated and infused with saline. Finally, the anchoring sutures at the IMF are secured. Once sutured in place, the BCP will provide complete inferior implant coverage in a similar fashion to the inferior expander coverage.

Support of the implant inferiorly with the BCP allows for the relaxed motion of the reconstructed breast, similar to the natural movement provided by the suspensory ligaments of Cooper in the breast. Because the BCP of the invention can be made in appropriate sizes, there is no excessive tension applied when the BCP is maneuvered over the implant to be sutured to the inferior border of the pectoralis major muscle. Because the BCP is not stretched and sutured under tension, the reconstructed breast lower pole is soft and relaxed but supports the implant inferiorly without tension to result in improved breast symmetry, superior cosmetic outcome, and reduced revision rate.

Although the foregoing invention has been described in some detail by way of illustration and example for purposes of clarity and understanding, it will be obvious to one of skill in the art that certain changes and modifications may be practiced within the scope of the appended claims.

Aspects and features of the present disclosure are set out in the following number of clauses which contain the subject matter of the clauses of the parent application as filed.
1. A prosthesis for mastopexy or breast reconstruction procedures comprising processed tissue material which, when implanted into a mammalian patient, undergoes controlled biodegradation occurring with adequate living cell replacement such that the original implanted prosthesis is remodeled by the patient's living cells; wherein the processed tissue material is derived from intestine or dermis; wherein the prosthesis has an arcuate or crescent shape; and wherein the prosthesis does not interfere with radiographic imaging.
2. A prosthesis for mastopexy or breast reconstruction procedures comprising two or more superimposed, chemically bonded layers of processed tissue material derived from tunica submucosa of small intestine which, when implanted into a mammalian patient, undergoes controlled biodegradation occurring with adequate living cell replacement such that the original implanted prosthesis is remodeled by the patient's living cells; and, wherein the prosthesis does not interfere with radiographic imaging.
3. A prosthesis for mastopexy or breast reconstruction procedures comprising two or more superimposed, chemically bonded layers of processed tissue material derived from tunica submucosa of small intestine which, when implanted into a mammalian patient, undergoes controlled biodegradation occurring with adequate living cell replacement such that the original implanted prosthesis is remodeled by the patient's living cells; and wherein the processed tissue material *consists essentially of* acellular telopeptide collagen, about 93% by weight dry; with less than about 5% dry weight glycoproteins, glycosaminoglycans, proteoglycans, lipids, non-collagenous proteins and nucleic acids such as DNA and RNA and is substantially free of cells and cellular debris.
4. A prosthesis for mastopexy or breast reconstruction procedures comprising two or more superimposed, chemically bonded layers of processed tissue material derived from tunica submucosa of small intestine which, when implanted into a mammalian patient, undergoes controlled biodegradation occurring with adequate living cell replacement such that the original implanted prosthesis is remodeled by the patient's living cells; and wherein the processed tissue material *consists essentially of* acellular telepeptide collagen, about 93% by weight dry; with less than about 5% dry weight glycoproteins, glycosaminoglycans, proteoglycans, lipids, non-collagenous proteins and nucleic acids such as DNA and RNA and is substantially free of cells and cellular debris; and, wherein the prosthesis does not interfere with radiographic imaging.
5. The prosthesis for use in mastopexy or breast reconstruction procedures of any preceding clause, wherein the prosthesis comprises between 2 and 10 bonded layers.
6. The prosthesis for use in mastopexy or breast reconstruction procedures of any preceding clause, wherein the prosthesis comprises between 3 and 5 bonded layers.
7. The prosthesis for mastopexy or breast reconstruction procedures of any preceding clause, wherein the bonded layers are crosslinked with a solution comprising 1-ethyl-3-(3-dimethylaminopropyl) carbodiimide hydrochloride.
8. The prosthesis for mastopexy or breast reconstruction procedures of any preceding clause, wherein the prosthesis additionally comprises a mesh arrangement running lengthwise across at least a portion of the prosthesis.
9. The prosthesis for mastopexy or breast reconstruction procedures of any preceding clause, wherein the prosthesis additionally comprises an arrangement of slits in parallel, staggered arrangement running lengthwise across at least a portion of the prosthesis.
10. A method for mastopexy treatment with reduction in breast volume, comprising:
   a. marking four points on the breast around the areola to determine the amount of skin necessary for both the external skin lining of the new breast and the excess skin in the periareaolar region for the dermal flap to be used for the internal skin lining
   b. de-epithelializing the flap to retain the central pedicle
   c. displace the breast subcutaneous down to the level of the pectoral fascia
   d. dissecting the skin on the bias in the upper hemisphere in order to progressively increase the thickness of the subcutaneous fat tissue close to the skin and
   e. resecting a central wedge of tissue and shortening the upper hemisphere ray
   f. dissecting the skin from the parenchymal tissue in the lower hemisphere of the breast
   g. optionally resecting a second central wedge of tissue in the lower hemisphere
   h. applying a mastopexy prosthesis over the dermal flap in the lower hemisphere to sling the underside of the breast
   i. suturing the mastopexy prosthesis to the pectoralis fascia to promote elevation and shape of the mammary cone
   j. suturing closed the external skin lining while fixing the areolar skin to the external skin lining
   k. dressing the breast in a supportive way that allows drainage of exudates.
11. A method for mastopexy treatment to lift breast tissue comprising
   a. Marking four points on the breast around the areola to determine the amount of skin necessary for both the external skin lining of the new breast and the excess skin in the periareaolar region for the dermal flap to be used for the internal skin lining
   b. De-epithelializing the flap to retain the central pedicle
   c. Displace the breast subcutaneous down to the level of the pectoral fascia
   d. Dissecting the skin on the bias in the upper hemisphere in order to progressively increase the thickness of the subcutaneous fat tissue close to the skin and
   e. Dissecting the skin from the parenchymal tissue in the lower hemisphere of the breast
   f. Applying a mastopexy prosthesis over the dermal flap in the lower hemisphere to sling the underside of the breast
   g. Suturing the mastopexy prosthesis to the pectoralis fascia to promote elevation and shape of the mammary cone
   h. Suturing closed the external skin lining while fixing the areolar skin to the external skin lining
   i. Dressing the breast in a supportive way that allows drainage of exudates.
12. A method for mastopexy treatment with augmentation (performed in either one or two operations) where the method comprises:
   a. inserting a breast implant either under the muscle in a submuscular pocket where the implant is large and the degree of sagging is greater, or under the breast gland in a subglandular pocket if the implant is small
   b. marking four points on the breast around the areola to determine the amount of skin necessary for both the external skin lining of the new breast and the excess skin in the periareaolar region for the dermal flap to be used for the internal skin lining
   c. de-epithelializing the flap to retain the central pedicle
   d. displace the breast subcutaneous down to the level of the pectoral fascia
   e. dissecting the skin on the bias in the upper hemisphere in order to progressively increase the thickness of the subcutaneous fat tissue close to the skin and
   f. dissecting the skin from the parenchymal tissue in the lower hemisphere of the breast
   g. applying a mastopexy prosthesis over the dermal flap in the lower hemisphere to sling the underside of the breast
   h. suturing the mastopexy prosthesis to the pectoralis fascia to promote elevation and shape of the mammary cone
   i. suturing closed the external skin lining while fixing the areolar skin to the external skin lining
   j. dressing the breast in a supportive way that allows drainage of exudates.

## Claims

1. A mastopexy prosthesis for use in a method for mastopexy treatment, wherein the method comprising the steps of:
(a) marking four points on the breast around the areola to determine the amount of skin necessary for both the external skin lining of the new breast and the excess skin in the periareaolar region for the dermal flap to be used for the internal skin lining
(b) de-epitheliializing the flap to retain the central pedicle c. displace the breast subcutaneous down to the level of the pectoral fascia
(d) dissecting the skin on the bias in the upper hemisphere in order to progressively increase the thickness of the subcutaneous fat tissue close to the skin and
(e) dissecting the skin from the parenchymal tissue in the lower hemisphere of the breast
(f) applying the mastopexy prosthesis over the dermal flap in the lower hemisphere to sling the underside of the breast
(g) suturing the mastopexy prosthesis to the pectoralis fascia to promote elevation and shape in the mammary cone
(h) suturing closed the external skin lining while fixing the areolar skin to the external skin lining
(i) dressing the breast in a supportive way that allows drainage of exudates.

2. The mastopexy prosthesis for use as claimed in claim 1, wherein the method of mastopexy treatment is a treatment to lift breast tissue.

3. The mastoexy prosthesis for use as claimed in claim 1, wherein the method for mastopexy treatment is a method of mastopexy treatment with a reduction in breast volume and further comprises the steps of:
after step (d), resecting a central wedge of tissue and shortening the upper hemisphere ray; and
after step (e), optionally resecting a second wedge of tissue in the lower hemisphere.

4. The mastopexy prosthesis for use as claimed in claim 1, wherein the method for mastopexy treatment is a method of mastopexy treatment with breast augmentation and further comprises the step of inserting a breast implant either under the muscle in a submuscular pocket where the implant is large and the degree of sagging is greater, or under the breast gland in a subglandular pocket if the implant is small.

5. The mastopexy prosthesis for use as claimed in claim 4, wherein the method is carried out in two operations.

6. The mastopexy prosthesis for use as claimed in any preceding claim, wherein the mastopexy prosthesis is bioremodelable and undergoes controlled biodegradation occuring concomitantly with remodeling and replacement by the patient's cells.

7. The mastopexy prosthesis for use as claimed in any preceding claim, wherein the mastopexy prosthesis comprises an arrangement of slits (50) in parallel, staggered arrangement comprising processed tissue material which, when implanted into a mammalian patient, undergoes controlled biodegradation occurring with adequate living cell replacement such that the original implanted prosthesis is remodeled by the patient's living cells; wherein the processed tissue material is derived from intestine or dermis; and wherein the prosthesis does not interfere with radiographic imaging, **characterised in that** the mastopexy prosthesis has an elongated arcuate or crescent shape and **in that** the arrangement of slits in parallel, staggered arrangement runs across at least a portion of the prosthesis in the lengthwise direction of the prosthesis.

8. The mastopexy prosthesis for use as defined in claim 7, wherein the processed tissue material comprises two or more superimposed, chemically bonded layers of processed tissue material derived from tunica submucosa of small intestine.

9. The mastopexy prosthesis for use as defined in claim 8, wherein the processed tissue material consists essentially of acellular telopeptide collagen, about 93% by weight dry; with less than about 5% dry weight glycoproteins, glycosaminoglycans, proteoglycans, lipids, non-collagenous proteins and nucleic acids such as DNA and RNA and is substantially free of cells and cellular debris.

10. The mastopexy prosthesis for use as defined in any one of claims 7 to 9, wherein the prosthesis comprises between 2 and 10 bonded layers.

11. The mastopexy prosthesis for use as defined in any one of claims 7 to 9, wherein the prosthesis comprises between 3 and 5 bonded layers.

12. The mastopexy prosthesis for use as defined in any one of claims 7 to 11, wherein the bonded layers are crosslinked with a solution comprising 1-ethyl-3-(3-dimethylaminopropyl) carbodiimide hydrochloride.

13. The mastopexy prosthesis for use as defined in any one of claims 7 to 12, wherein the prosthesis additionally comprises a mesh ratio of between 1:1 and 3:1.

14. The mastopexy prosthesis for use as defined in any one claims 7 to 13, wherein the prosthesis additionally comprises a mesh ratio of between 1:1 and 1.5:1.
